(19) Europäisches Patentamt
European Patent Office
Office européen des brevets

(11) Publication number: **0 146 258 A2**

(12) **EUROPEAN PATENT APPLICATION**

(21) Application number: **84307721.5**

(22) Date of filing: **08.11.84**

(51) Int. Cl.[4]: **C 07 D 295/08**, C 07 D 295/14, C 07 D 211/22, C 07 D 211/46, C 07 D 213/20, C 07 D 277/22, C 07 D 233/02, C 07 D 453/02, A 61 K 31/395

(30) Priority: **10.11.83 JP 209936/83**

(43) Date of publication of application: **26.06.85 Bulletin 85/26**

(84) Designated Contracting States: **AT BE CH DE FR GB IT LI LU NL SE**

(71) Applicant: **ONO PHARMACEUTICAL CO., LTD., No. 14, Doshomachi 2-chome Higashi-ku, Osaka-shi Osaka (JP)**

(72) Inventor: **Arai, Yoshinobu, 1-5-8-505 Wakayama-dai, Shimamoto-cho Mishima-gun Osaka (JP)**
Inventor: **Hamanaka, Nobuyuki, 11-38 Hiroshiki Ohyamazaki-cho, Otokuni-gun Kyoto (JP)**
Inventor: **Miyamoto, Tsumoru, 1-40, Tonomoriyama, Jyouyou-shi Kyoto (JP)**

(74) Representative: **Pearce, Anthony Richmond et al, Marks & Clerk Alpha Tower Suffolk Street, Queensway Birmingham B1 1TT (GB)**

(54) **Glycerol derivatives.**

(57) Glycerol derivatives of the general formula:

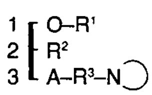 (I)

[wherein A represents an oxygen atom or a carbonyloxy group (wherein the carbonyl group should be attached to a group $R^3$ and the oxa group should be attached to the carbon atom at the third position of the glycerol skeleton), $R^1$ represents an alkyl group of 6 to 22 carbon atoms, or an alkyl group of 2 to 5 carbon atoms being substituted by a phenyl group, $R^2$ represents a hydroxy group, an amino group, an azido group, an alkyl group of 1 to 20 carbon atoms, an alkoxy group of 1 to 20 carbon atoms, an alkenyl group of 2 to 20 carbon atoms, an alkenyloxy group of 3 to 20 carbon atoms, or a group of the formula:

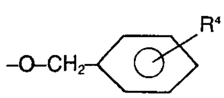 ,

$-OCOR^5$, $-OCONHR^5$, $-NHCOR^5$, $-NHCOOR^5$ or $NHCONHR^5$, in which $R^4$ represents a hydrogen atom, a halogen atom, an alkyl group of 1 to 4 carbon atoms or an alkoxy group of 1 to 4 carbon atoms and $R^5$ represents an alkyl group of 1 to 10 carbon atoms or a phenyl group, $R^3$ represents an alkylene group of 1 to 12 carbon atoms ans 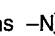 represents a heterocyclic group, with the proviso that, when A represents the carbonyloxy group, $R^2$ does not represent an amino group], and non-toxic acid addition salts thereof, possess antagonistic effect on PAF, hypotensive effect like PAF, inhibitory effect on phospholipase and inhibitory effect on growth of tumour cell or induction of differentiation, and are, therefore, useful as a platelet aggregation inhibitor, anti-asthmatic agent, anti-allergic agent, anti-inflammatory agent, hypotensive agent and anti-tumour agent for mammals.

EP 0 146 258 A2

# D E S C R I P T I O N

## GLYCEROL DERIVATIVES

The present invention is concerned to new glycerol derivatives. More particularly, this invention is concerned to new glycerol derivatives having antagonistic effect on platelet activating factor (abbreviated as PAF hereafter), hypotensive effect like PAF, inhibitory effect on phospholipase and further, inhibitory effect on growth of tumour cell or induction of differentiation, and, furthermore, this invention is concerned to the process for their preparation and pharmaceutical composition comprising them as active ingredients.

Recently, the study on the release reaction of active amine dependent on leukocyte from platelet, has been energetically carried out. As a result, a substance strongly inducing platelet aggregation was discovered and it was named as PAF [cf. J. Immunol., 106, 1244 (1971), J. Exp. Med., 136, 1356 (1972) and Nature, 249, 581 (1974)]. Thereafter, it was confirmed that PAF exists in various animals including human beings. In 1979, its chemical structure was identified [cf. J. Biol. Chem., 254, 9355 (1979) and C. R. Acad. Sci., Série D (Paris), 289, 1017 (1979)] and has turned out to be a mixture of two alkylphospholipids of the following general formula wherein n is 15 and 17 [cf. J. Biol. Chem., 255, 5514 (1980)]:

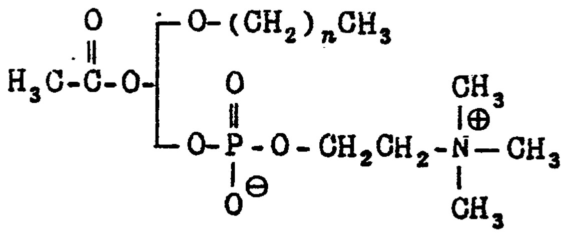

(wherein n represents 15 or 17).

It was found that PAF has not only strong secretory effect on platelet aggregation known at that time, but also powerful hypotensive effect and bronchoconstricting effect as physiological effect. PAF is considered to be one of platelet aggregating factors in human beings, and further, one of substances inducing allergy and inflammation. Accordingly, there is a great possibility that compounds having antagonistic effect on PAF (inhibitory effect on PAF) may become an unprecedented platelet aggregation inhibitor, anti-asthmatic agent, anti-allergic agent or anti-inflammatory agent. Furthermore, PAF is considered to become a hypotensive agent without inhibitory effect on platelet aggregation by selecting hypotensive effect out of various effect.

Being interested in physiological effect of PAF, we have carried out widespread investigations in order to discover compounds similar to PAF in their chemical structure and possessing antagonistic effect on PAF or hypotensive effect like PAF. We have found that the above purpose can be accomplished by glycerol derivatives which are replaced the hydroxy group at the first position of the glycerol skeleton by an alkoxy group and

are replaced the hydroxy group at the third position thereof by an alkoxy group substituted by a primary, secondary, tertiary or quaternary amine, preferably tertiary or quaternary amine.

For example, our European Patent Publication No. 94586 discloses the compounds of the general formula:

$$\left[\begin{array}{l} A^{1}-R^{11} \\ B^{1}-R^{21} \\ D^{1}-R^{31}-R^{41} \end{array}\right.$$

(wherein $A^1$ is -O- or -S-, $B^1$ is -O- or -S-, $D^1$ is -O- or -OCO-, $R^{11}$ is alkyl, $R^{21}$ is hydrogen, benzyl, acyl, N-substituted amido when $B^1$ is -O-, or $R^{21}$ is hydrogen, alkyl, benzyl or alkoxy(thiocarbonyl) when $B^1$ is -S-, $R^{31}$ is alkylene and $R^{41}$ is amino, mono- or dialkyl-substituted amino or trialkyl-substituted ammonium), and
our European Patent Publication No. 109255 discloses the compounds of the general formula:

$$\left[\begin{array}{l} O-R^{12} \\ R^{22} \\ A^{2}-R^{32}-R^{42} \end{array}\right.$$

(wherein $A^2$ is -O- or -OCO-, $R^{12}$ is alkyl, $R^{22}$ is azido, amino, acylamino, alkoxycarbonylamino or ureido, $R^{32}$ is alkylene and $R^{42}$ is amino, mono- or dialkyl-substituted amino or trialkyl-substituted ammonium).

Now, we have found that by replacing an alkyl-substituted amino group at the end of the third position of the glycerol skeleton hereinbefore described, by a heterocyclic group containing nitrogen atom(s), new glycerol compounds are more active in antagonistic effect on PAF than glycerol compounds previously proposed, having completed this invention.

On the other hand, glycerol compounds similar to those of the present invention in chemical structure are disclosed in the German Patent Publication No. 2835369 (abbreviated as DE-2835369 hereafter). Broad scope of compounds is disclosed therein and the compounds of the general formula:

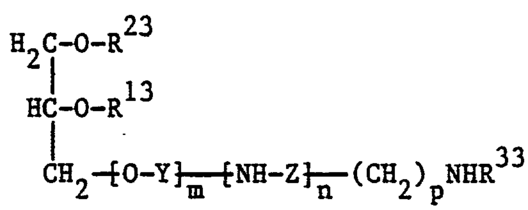

(A)

(wherein $R^{13}$ and $R^{23}$ each represents a normal alkyl or alkenyl group of 12 to 20 carbon atoms and Y and Z each represents an alkylene group of 2 to 4 carbon atoms or ..., $R^{33}$ represents a hydrogen atom, an alkyl group of 2 to 4 carbon atoms or ..., m, n and p each represents zero or 1 and the total number of m, n and p is zero or 1) are most related to our invention (the definitions not related to our invention are removed from the above description).

As understood from the above definition, $-NHR^{33}$ in formula (A) in DE-2835369 represents only a primary or secondary amine, while 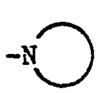 in the general formula (I) hereinafter

described, of the present invention represents a heterocyclic group containing nitrogen atom(s).

Furthermore, the distinguished difference between the present invention and the invention disclosed in DE-2835369 is utility of glycerol compounds. That is, compounds of the present invention have antagonistic effect on PAF and hypotensive effect like PAF and, therefore, are used as platelet aggregation inhibitor, anti-asthmatic agent, anti-allergic agent and anti-inflammatory agent, whereas compounds in DE-2835369 have stimulating activity of production of interferon and are useful for prophylaxis of viral disease.

As discussed above, the present invention is remarkably different from the invention disclosed in DE-2835369 in chemical structure and their utility. The present invention is not anticipated and not obvious from that of DE-2835369.

It, furthermore, has been found that the compounds of the present invention of the general formula (I) have inhibitory effect on phospholipase $A_2$ and phospholipase C. Phospholipase $A_2$ and phospholipase C dependent on calcium in a living body are concerned in the mechanism for the release of arachidonic acid from phospholipids. It is known that arachidonic acid is released by physiological stimulus and then becomes incorporated into the metabolic routes of prostaglandins and is finally metabolized to thromboxane $A_2$ having a strong effect on platelet aggregation, to other prostaglandins or to various leukotrienes being a significant bronchostrictor in an asthmatic paroxysm. Accordingly, it is considered that compounds having an

inhibitory effect on phospholipase $A_2$ and phospholipase C, which are concerned in the mechanism for the release of arachidonic acid, can become a platelet aggregation inhibitor, anti-asthmatic agent, anti-allergic agent or anti-inflammatory agent, based on the inhibition of phospholipase $A_2$ and phospholipase C, having a different mechanism from PAF antagosist and further, being different from conventional non-steroid anti-inflammatory agents represented by aspirin based on the inhibition of cyclooxygenase.

Furthermore, the compounds of the present invention of the general formula (I) show inhibitory effect on growth of tumour cell and induction of differentiation and, therefore, may be useful as anti-tumour agents.

Accordingly, the present invention is concerned to new glycerol derivatives of the general formula:

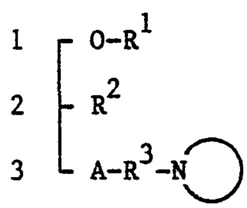

(I)

[wherein A represents an oxygen atom or a carbonyloxy group (i.e. -O-CO- group (wherein the carbonyl group should be attached to a group $R^3$ and the oxa group should be attached to the carbon atom at the third position of the glycerol skeleton)), $R^1$ represents an alkyl group of 6 to 22 carbon atoms, or an alkyl group of 2 to 5 carbon atoms being substituted by a phenyl group,

$R^2$ represents a hydroxy group, an amino group ($-NH_2$ group), an azido group ($-N_3$ group), an alkyl group of 1 to 20 carbon atoms, an alkoxy group of 1 to 20 carbon atoms, an alkenyl group of 2 to 20 carbon atoms, an alkenyloxy group of 3 to 20 carbon atoms, or a group of the formula: $-O-CH_2-$ 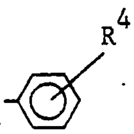 , $-OCOR^5$, $-OCONHR^5$, $-NHCOR^5$, $-NHCOOR^5$ or $-NHCONHR^5$, in which $R^4$ represents a hydrogen atom, a halogen atom, an alkyl group of 1 to 4 carbon atoms or an alkoxy group of 1 to 4 carbon atoms and $R^5$ represents an alkyl group of 1 to 10 carbon atoms or a phenyl group, $R^3$ represents an alkylene group of 1 to 12 carbon atoms and -N 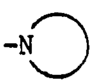 represents a heterocyclic group, with the proviso that, when A represents the carbonyloxy group, $R^2$ does not represent an amino group],

and non-toxic acid addition salts thereof.

Throughout the specification including claims, the term "alkyl", "alkenyl", "alkylene", and further "alkyl" and "alkenyl" in alkoxy and alkenyloxy groups, respectively, mean a straight- or branched-chain alkyl, alkenyl and alkylene group.

Examples of the alkyl group of 6 to 22 carbon atoms represented by $R^1$ in the general formula (I), are hexyl, heptyl, octyl, nonyl, decyl, undecyl, dodecyl, tridecyl, tetradecyl, pentadecyl, hexadecyl, heptadecyl, octadecyl, nonadecyl, eicosyl, heneicosyl and docosyl group and isomers thereof. An alkyl group of 14 to 22 carbon atoms is preferred and hexadecyl group is most preferred.

Examples of the alkyl group of 2 to 5 carbon atoms substituted by a phenyl group, represented by $R^1$ are ethyl, propyl, butyl and pentyl group and isomers thereof replaced an optional hydrogen atom by a phenyl group.

Examples of the alkyl group of 1 to 20 carbon atoms represented by $R^2$ in the general formula (I), are methyl, ethyl, propyl, butyl, pentyl, hexyl, heptyl, octyl, nonyl, decyl, undecyl, dodecyl, tridecyl, tetradecyl, pentadecyl, hexadecyl, heptadecyl, octadecyl, nonadecyl and eicosyl group and isomers thereof. An alkyl group of 1 to 6 carbon atoms is preferred.

Examples of the alkenyl group of 2 to 20 carbon atoms represented by $R^2$, are an alkenyl group having a carbon-carbon double bond at any one position of the alkyl group hereinbefore illustrated (except for methyl group). An alkenyl group of 2 to 6 carbon atoms (e.g. allyl group) is preferred.

Examples of the alkoxy group of 1 to 20 carbon atoms represented by $R^2$ are methoxy, ethoxy, propoxy, butoxy, pentyloxy, hexyloxy, heptyloxy, octyloxy, nonyloxy, decyloxy, undecyloxy, dodecyloxy, tridecyloxy, tetradecyloxy, pentadecyloxy, hexadecyloxy, heptadecyloxy, octadecyloxy, nonadecyloxy and eicosyloxy group and isomers thereof. An alkoxy group of 1 to 6 carbon atoms is preferred.

Examples of the alkenyloxy group of 3 to 20 carbon atoms represented by $R^2$, are an alkenyloxy group having a carbon-carbon double bond at the ß-position or at any one position thereafter of the alkoxy group hereinbefore

illustrated (except for methoxy and ethoxy group). An alkenyloxy group of 3 to 6 carbon atoms (e.g. allyloxy group) is preferred.

Examples of the substituent $R^4$ in the formula:

$-O-CH_2-$ 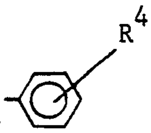 represented by $R^2$ are hydrogen atom and

fluorine, chlorine, bromine and iodine atom, and methyl, ethyl, propyl, butyl, methoxy, ethoxy, propoxy and butoxy group and isomers thereof. Preferably $R^4$ represents hydrogen atom, chlorine atom, methyl group and methoxy group, and most preferably $R^4$ is a hydrogen atom.

Examples of the alkyl group of 1 to 10 carbon atoms represented by $R^5$ in the formulae: $-OCOR^5$, $-OCONHR^5$, $-NHCOR^5$, $-NHCOOR^5$ and $-NHCONHR^5$ represented by $R^2$, are methyl, ethyl, propyl, butyl, pentyl, hexyl, heptyl, octyl, nonyl and decyl group and isomers thereof. An alkyl group of 1 to 6 carbon atoms, i.e. methyl, ethyl, propyl, isopropyl, butyl, isobutyl, sec-butyl, tert-butyl group, and further pentyl and hexyl group and isomers thereof is preferred. A phenyl group as $R^5$ is also preferred.

Any $R^2$ group is preferred and especially preferred $R^2$ group is hydroxy group, amino group, azido group, or alkyl or alkoxy group of 1 to 6 carbon atoms, or a group of the

formula: $-O-CH_2-$ 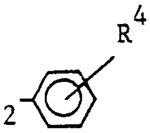 , $-OCOR^5$, $-NHCOR^5$, $-NHCOOR^5$ or $-NHCONHR^5$ (wherein the various symbols are as hereinbefore

defined).

Examples of the alkylene group of 1 to 12 carbon atoms represented by $R^3$ in the general formula (I), are methylene, ethylene, trimethylene, tetramethylene, pentamethylene, hexamethylene, heptamethylene, octamethylene, nonamethylene, decamethylene, undecamethylene and dodecamethylene group and isomers thereof, and any group is preferred.

A heterocyclic group represented by 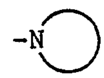 in the general formula (I) means such a heterocyclic group as meets the following requirements:

(1) the heterocyclic group consists of 4 to 10 atoms,

(2) the group consists of (i) one nitrogen atom, or one nitrogen atom and other one or two nitrogen, oxygen or sulfur atom (with the proviso that the total number of hetero atoms is up to 3), and (ii) at least two carbon atoms,

(3) the group is single ring or bridged bicyclo ring,

(4) the group has or does not have double bond(s) in the ring, and is aromatic or non-aromatic ring,

(5) the group may be substituted by 1 to 5 alkyl groups of 1 to 6 carbon atoms (e.g. methyl, ethyl, propyl, butyl, pentyl and hexyl group and isomers thereof, preferably methyl group) at any optional position in the ring (preferably nitrogen atom or carbon atom), and

(6) the group may be substituted by 1 or 2 hydroxy groups at any optional position in the ring (preferably carbon atom) when $R^2$ represents a hydroxy group, an amino group, an azido group, an alkyl group of 1 to 20 carbon atoms, an alkoxy

group of 1 to 20 carbon atoms, an alkenyl group of 2 to 20 carbon atoms, an alkenyloxy group of 3 to 20 carbon atoms, or a group of the formula: $-O-CH_2-$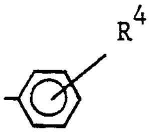, $-NHCOR^5$, $-NHCOOR^5$ or $-NHCONHR^5$, in which $R^4$ and $R^5$ are as hereinbefore defined.

Further, these heterocyclic groups may form salts with suitable anions hereinafter described when quaternary ammonium ion(s) exist(s) in the ring _per se_ or when nitrogen atom(s) in the ring is (are) substituted by alkyl group(s) to form quaternary ammonium ion(s).

Examples of the heterocyclic group are, for example,

(1) 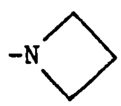 (1-azetidinyl group), 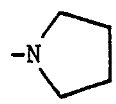 (1-pyrrolidinyl group), 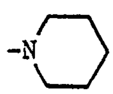 (piperidino group), 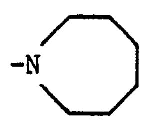 (hexamethyleneimino group) and 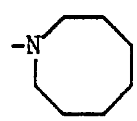 (heptamethylene-imino group),

(2) 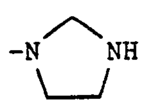 (1-imidazolidinyl group) and 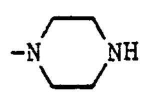 (1-piperazinyl group) and isomers thereof,

(3) 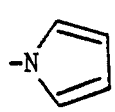 (1-pyrrolyl group), 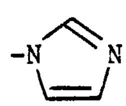 (1-imidazolyl group),

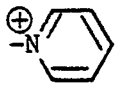 (1-pyridinio group) and 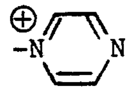 (1-pyrazinio group)

and isomers thereof and partially saturated group thereof,

(4) 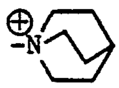 (1-quinuclidinio group) and 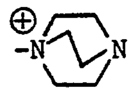 (triethylene-

diamin-1-io group) and isomers thereof,

(5) 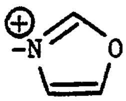 (3-oxazolio group) and 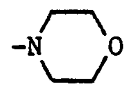 (morpholino group)

and isomers thereof and partially or completely saturated

or unsaturated groups thereof, and

(6) 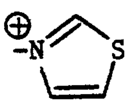 (3-thiazolio group)and isomers thereof and partially

or completely saturated groups thereof; and N-alkyl

substituted groups thereof; and hydroxy-substituted group

thereof.

Preferable heterocyclic groups are 1-pyrrolidinyl, piperidino, hexamethyleneimino, 1-piperazinyl, 1-imidazolyl, 1-pyridinio, 1-quinuclidinio, triethylenediamin-1-io, morpholino or 3-thiazolio group, or N-alkyl substituted groups thereof, or hydroxy-substituted groups thereof.

When the heterocyclic group represented by 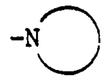 is a quaternary ammonium ion, the anion which forms salts with such ammonium ion means the anion of a pharmaceutically-acceptable non-toxic inorganic or organic acid, and examples of them are the anion of an inorganic acid such as hydrochloric acid, hydrobromic acid, hydroiodic acid, sulfuric acid, phosphoric acid, nitric acid, or the anion of an organic acid such as acetic acid, lactic acid, tartaric acid, benzoic acid, citric acid, methanesulfonic acid, ethanesulfonic acid, benzenesulfonic acid, toluenesulfonic acid, isethionic acid. Preferable anion is a halogen ion, i.e. chlorine, bromine and iodine ions, and the anion of methanesulfonic acid and p-toluenesulfonic acid.

The compounds of the general formula (I) have at least one asymmetric carbon (the carbon atom at the second position of the glycerol skeleton). Furthermore, when the alkyl moiety of various substituents represented by $R^1$, $R^2$ and $R^3$ represents a branched-chain, there is a possibility that other asymmetric centers are occurred. However the compounds of the general formula (I) in the present invention are concerned with all isomers and mixtures thereof, which are formed by asymmetric carbons.

According to a feature of the present invention, compounds of the general formula (I), wherein 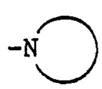 represents a heterocyclic group having quaternary ammonium ion(s) in the ring per se (e.g. 1-pyridinio, 1-quinuclidinio, 3-thiazolio, triethylenediamin -1-io group etc.), or having quaternary ammonium ion(s) by substituting nitrogen atom(s) in the ring by

alkyl group(s) (e.g. N-methyl-1-pyrrolidinio, N-methylmorpholinio group etc.), i.e. compounds of the general formula:

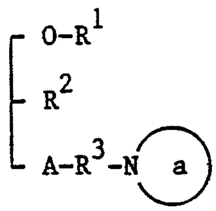

(IA)

(wherein 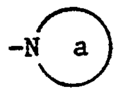 represents a heterocyclic group having quaternary ammonium ion(s) in the ring *per se* or having quaternary ammonium ion(s) by substituting nitrogen atom(s) in the ring by alkyl group(s), and the other symbols are as hereinbefore defined) may be prepared by reacting a compound of the general formula:

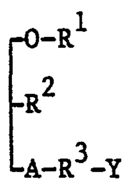

(II)

(wherein Y represents a halogen atom (preferably chlorine or bromine atom), an alkylsulfonyloxy group or a substituted or unsubstituted arylsulfonyloxy group (preferably mesyloxy or tosyloxy group), and the other symbols are as hereinbefore defined) with a tertiary amine of the general formula:

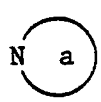

(IIIa)

(wherein 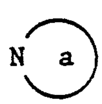 is as hereinbefore defined), for example,

pyridine, quinuclidine, thiazole, triethylenediamine, N-methylpyrrolidine, N-methylmorpholine etc., and then, if desired, performing salt-exchanging reaction.

The reaction between compounds of the general formula (II) and those of the general formula (IIIa) may be carried out in an appropriate inert organic solvent such as a lower alkanol, e.g. methanol, ethanol, isopropanol, or dimethylformamide, or a mixture of two or more of them at a temperature from ambient to a reflux temperature of the reaction mixture for one hour to several days. It is preferable to use 2.0 mols or more of the compound of the general formula (IIIa) to one mol of the compound of the general formula (II). In this reaction, quaternary ammonium ions may be produced to form salt with anions represented by $\overset{\ominus}{Y}$ (in which $\overset{\ominus}{Y}$ is as hereinbefore defined).

Salts formed by this reaction may be converted into salts of anions of acids other than anions represented by $\overset{\ominus}{Y}$ by salt-exchanging reaction. That is, it is performed (i) by neutralizing of a salt with $\overset{\ominus}{Y}$ ion with an aqueous solution of sodium hydroxide, and then reacting with a desirable inorganic or organic acid, or (ii) by ion-exchange resin.

According to a further feature of the present invention, compounds of the general formula (I), wherein 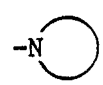 represents a heterocyclic group having tertiary amine(s) (e.g. 1-pyrrolidinyl, 1-piperazinyl, morpholino group etc.), i.e. compounds of the general formula:

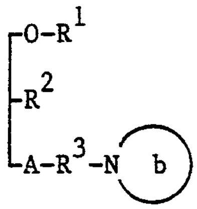

(IB)

,

(wherein 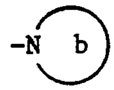 represents a heterocyclic group having tertiary amine(s) and the other symbols are as hereinbefore defined) may be prepared by the following methods.

Compounds of the general formula (IB) may be prepared by:

(1) reacting a compound of the general formula:

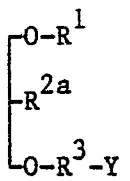

(IIA)

(wherein $R^{2a}$ represents a hydroxy group, an amino group, an azido group, an alkyl group of 1 to 20 carbon atoms, an alkoxy group of 1 to 20 carbon atom, an alkenyl group of 2 to 20 carbon atoms, an alkenyloxy group of 3 to 20 carbon atoms, or a group of the formula: $-O-CH_2-$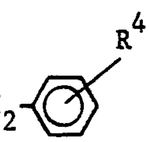, $-OCONHR^5$, $-NHCOR^5$, $-NHCOOR^5$ or $-NHCONHR^5$, in which $R^4$ and $R^5$ are as hereinbefore defined, and the other symbols are as hereinbefore defined) with a secondary amine of the general formula:

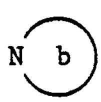

(IIIb)

(wherein 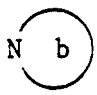 is as hereinbefore defined), for example, pyrrolidine, piperazine, morpholine etc. to obtain a compound of the general formula:

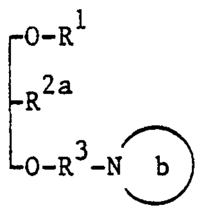

(IB-1)

(wherein the various symbols are as hereinbefore defined),

(2) esterifying a compound of the general formula:

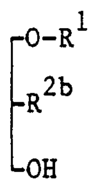

(IV)

(wherein $R^{2b}$ represents an azido group, an alkyl group of 1 to 20 carbon atoms, an alkoxy group of 1 to 20 carbon atoms, an alkenyl group of 2 to 20 carbon atoms, an alkenyloxy group of 3 to 20 carbon atoms, or a group of the formula: 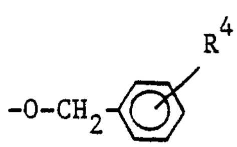 , $-NHCOR^5$, $-NHCOOR^5$ or $-NHCONHR^5$, in which $R^4$ and $R^5$ are as hereinbefore defined, and $R^1$ is as hereinbefore defined) with an acid of the general formula:

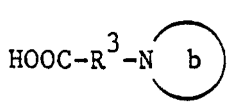

(V)

(wherein the various symbols are as hereinbefore defined) or a reactive derivative thereof to obtain a compound of the general formlua:

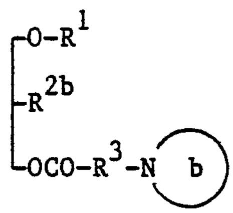

(IB-2)

(wherein the various symbols are as hereinbefore defined),

(3) reactive elimination of the benzyl group in a compound of the general formula:

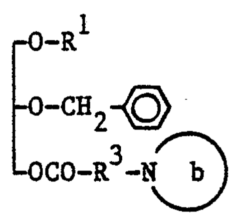

(IB-21)

(wherein the various symbols are as hereinbefore defined) to obtain a compound of the general formula:

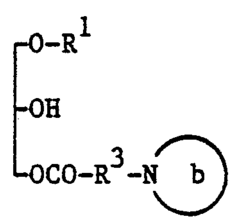

(IB-3)

(wherein the various symbols are as hereinbefore defined),

(4) esterifying a compound of the general formula:

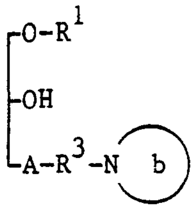

(IB-4)

(wherein the various symbols are as hereinbefore defined) with an acid of the general formula:

$HOOC-R^5$ (VI)

(wherein $R^5$ is as hereinbefore defined) or a reactive derivative thereof to obtain a compound of the general formula:

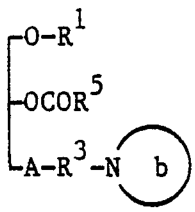

(IB-5)

(wherein the various symbols are as hereinbefore defined), or

(5) reacting a compound of the general formula:

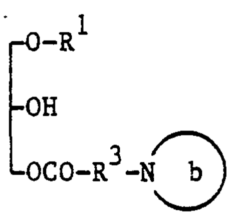

(IB-3)

(wherein the various symbols are as hereinbefore defined) with an isocyanate of the general formula:

$O=C=N-R^5$ (VII)

(wherein $R^5$ is as hereinbefore defined) to obtain a compound of the general formula:

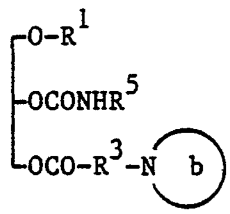

(IB-6)

(wherein the various symbols are as hereinbefore defined).

The reaction (1) may be carried out by methods hereinbefore described for the reaction of compounds of the general formula (II) with those of the general formula (IIIa). By this reaction a tertiary amine may be formed.

The esterification (2) may be carried out by known methods, for example, in an inert organic solvent such as ethyl acetate, benzene, diethyl ether, chloroform, methylene chloride or tetrahydrofuran or a mixture of two or more of them at a temperature from -10°C to 50°C (preferably at a temperature from ambient to 0°C). Examples of the reactive derivatives of an acid of the general formula (V) are an acid halide such as acyl chloride or acyl bromide, an acid anhydride, a mixed acid anhydride with pivaloyl chloride, isobutyryl chloride or isobutyl chloroformate, or an active ester with dicyclohexylcarbodiimide (DCC). Preferably, the reaction may be carried out in the presence of a base such as pyridine or 4-dimethylaminopyridine.

The elimination (3) of the benzyl group may be carried out, for example, under an atmosphere of hydrogen in the presence of a hydrogenation catalyst such as palladium on carbon or

palladium black in an inert organic solvent, e.g. a lower alkanol such as methanol or ethanol, or ethyl acetate, or a mixture of two or more of them, preferably in the presence of an acid such as acetic acid, hydrochloric acid, hydroiodic acid or methanesulfonic acid, at a temperature from ambient to the reflux temperature of the reaction mixture.

The esterification (4) may be carried out by methods hereinbefore described for the esterification (2).

The reaction (5) may be carried out by reacting with an isocyanate of the general formula (VII) in an inert organic solvent, e.g. ethyl acetate, benzene, hexane, diethyl ether, tetrahydrofuran, chloroform, methylene chloride or carbon tetrachloride, or a mixture of two or more of them, in the presence of a tertiary amine such as pyridine or triethylamine at a temperature from 0°C to 50°C.

Compounds of the general formula (IB) which is prepared by these reactions, may be converted into quaternary ammonium salts by alkylation. Such alkylation may be carried out by reacting with an alkyl halide such as methyl iodide in a lower alkanol such as methanol, ethanol or isopropanol at a temperature from ambient to the reflux temperature of the reaction mixture. In this case, the salts with a halogen ion may be formed. Compounds of the general formula (IB) wherein $R^2$ is an amino group can not be converted into quaternary ammonium salts by such alkylation. Therefore, it is necessary to react compounds of the general formula (II) with those of the general formula (IIIa) which are previously alkylated, to obtain desirable quaternary

ammonium salts.

Furthermore, salts which is prepared by alkylation hereinbefore described, may be converted into salts of anions of acids other than halogen ions by salt-exchanging reaction hereinbefore described.

The heterocyclic compounds of the general formula (IIIa) and (IIIb) and N-alkyl substituted compounds thereof, and compounds of the general formula (V), (VI) and (VII), which all are used in the reactions hereinbefore described, are on the market as known compounds or may be easily prepared by methods known _per se_.

In compounds of the general formula (II) used as starting materials, compounds wherein $R^2$ represents a hydroxy group or a group of the formula: $-O-CH_2-$ 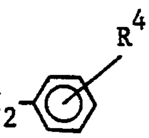 , in which $R^4$ is as hereinbefore defined (hereinafter depicted in the general formula (IIB-1) to (IIB-8)) are partly described in any one of the specifications of the Japanese Patent Publication Nos. 58-198445, 59-20255, 59-31738, 59-88447, 59-110663 and 59-112961, and the European Patent Publication Nos. 94586 and 109255 (abbreviated as the reference "A" hereafter), and may be prepared by the series of reactions depicted schematically below in Scheme I, wherein X represents a halogen atom, T represents an alkylsulfonyl group or a substituted or unsubstituted arylsulfonyl group (preferably mesyl or tosyl group), THP represents a tetrahydropyran-2-yl group and the other symbols are as hereinbefore defined.

Scheme I

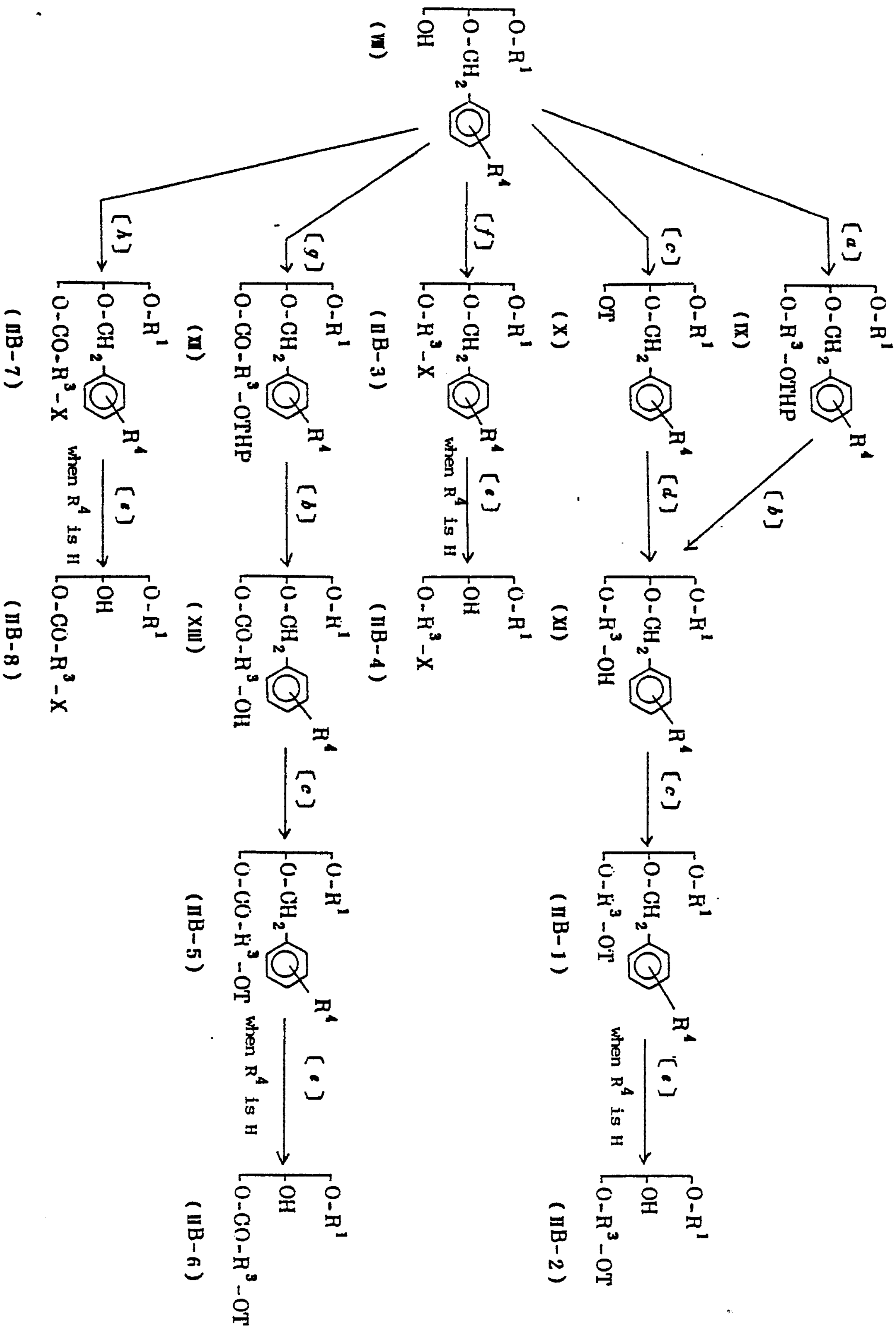


(VIII) (IX) (X) (XI) (IIB-1) (IIB-2) (IIB-3) (IIB-4) (XII) (XIII) (IIB-5) (IIB-6) (IIB-7) (IIB-8)

In Scheme I, each step can be effected using methods known *per se*. For example, step (a) may be carried out by reacting a compound of the general formula (VIII) with a compound of the general formula:

$$Q\text{-}R^3\text{-}OTHP \qquad (XIV)$$

(wherein Q represents a halogen atom, or an alkylsulfonyloxy group or a substituted or unsubstituted arylsulfonyloxy group (preferably mesyloxy or tosyloxy group) and the other symbols are as hereinbefore defined) in the presence of an alkali metal hydride such as sodium hydride in an inert organic solvent such as dimethylformamide or tetrahydrofuran at a temperature from ambient to 100°C.

Step (b) is the elimination of THP group, and may be carried out by using a mixture of tetrahydrofuran, acetic acid and water, a mixture of p-toluenesulfonic acid and anhydrous methanol, or a mixture of diluted hydrochloric acid and tetrahydrofuran, at room temperature or with heating.

Step (c) may be carried out by reacting a compound of the general formula (VIII) with an alkylsulfonyl chloride such as mesyl chloride or with an arylsulfonyl chloride such as tosyl chloride at a temperature from -30°C to 50°C, (i) in the presence of a tertiary amine such as pyridine or triethylamine in an inert organic solvent such as methylene chloride, or (ii) in pyridine.

Step (d) and (f) may be carried out by methods

hereinbefore described for step (a) by using a compound of the general formula:

$$HO-R^3-OH \qquad (XV)$$

and

$$Q-R^3-X \qquad (XVI)$$

(wherein the various symbols are as hereinbefore defined), respectively, instead of a comound of the general formula (XIV).

Step (e) may be carried out by reductive elimination of the benzyl group attached to the second position of a comound of the general formula (IIB-1), (IIB-3), (IIB-5) and (IIB-7) wherein $R^4$ is a hydrogen atom, by methods hereinbefore described for the reaction (3).

Step (g) and (h) may be carried out by esterifying a compound of the general formula (VIII) with an acid of the general formula:

$$HOOC-R^3-OTHP \qquad (XVII)$$

and

$$HOOC-R^3-X \qquad (XVIII)$$

(wherein the various symbols are as hereinbefore defined) or a reactive derivative thereof, respectively, by method hereinbefore described for the esterification (2).

In compounds of the general formula (II) used as starting materials, compounds wherein $R^2$ represents an alkyl

group of 1 to 20 carbon atoms, an alkoxy group of 1 to 20 carbon atoms, an alkenyl group of 2 to 20 carbon atoms, an alkenyloxy group of 3 to 20 carbon atoms, or a group of the formula: $-OCOR^5$ or $-OCONHR^5$, in which $R^5$ is as hereinbefore defined (hereinafter depicted in the general formula (IIC-1) to (IIC-13)) are partly described in any one of the reference "A", and may be prepared by the series of reactions depicted schematically below in Scheme II and III, wherein $R^6$ represents an alkyl group of 1 to 20 carbon atoms or an alkenyl group of 2 to 20 carbon atoms, $R^7$ represents an alkyl group of 1 to 20 carbon atoms or an alkenyl group of 3 to 20 carbon atoms and the other symbols are as hereinbefore defined.

Scheme II

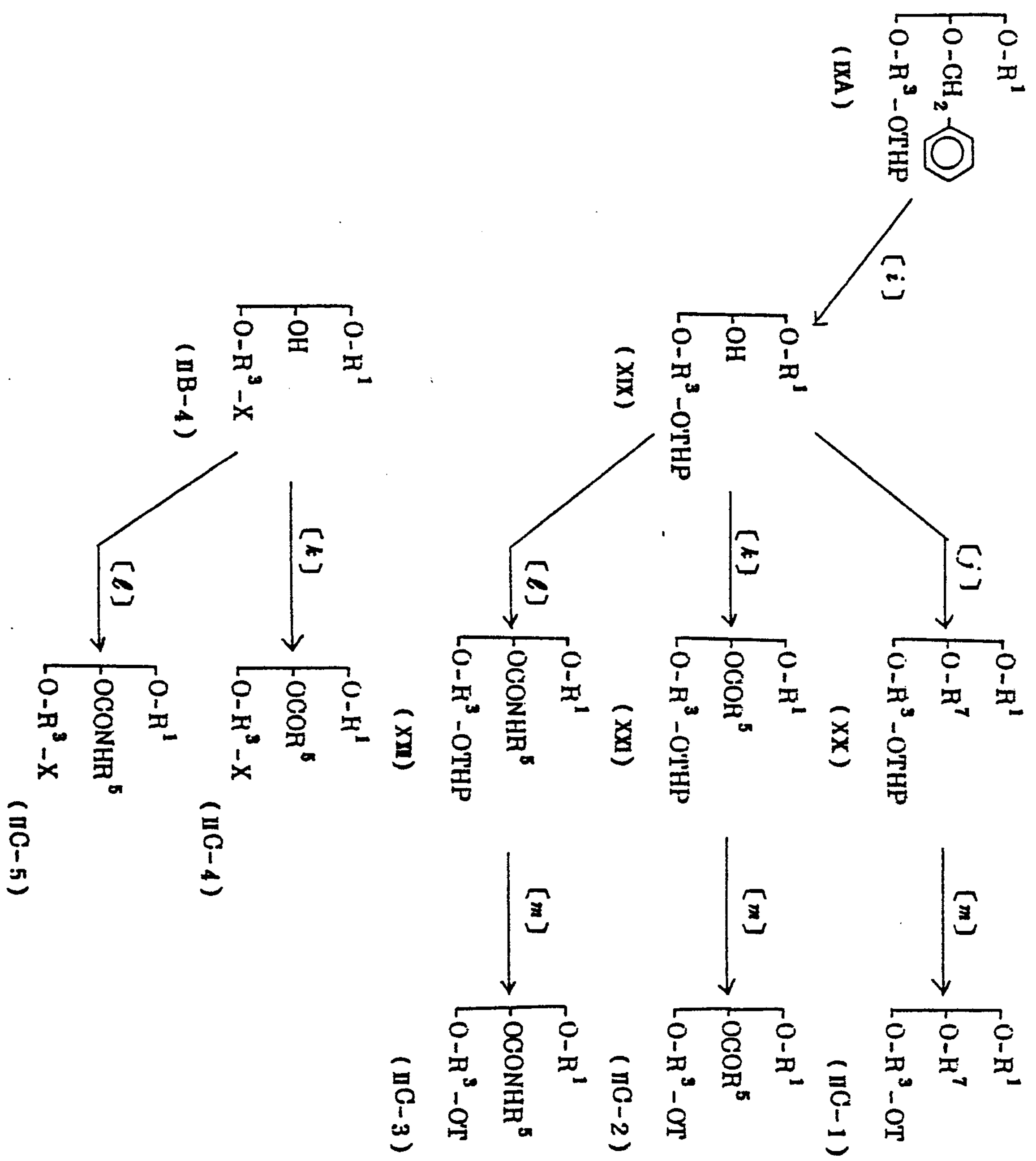


(IXA)
(XIX)
(XX)
(XXI)
(XXII)
(IIB-4)
(IIC-1)
(IIC-2)
(IIC-3)
(IIC-4)
(IIC-5)

Scheme III

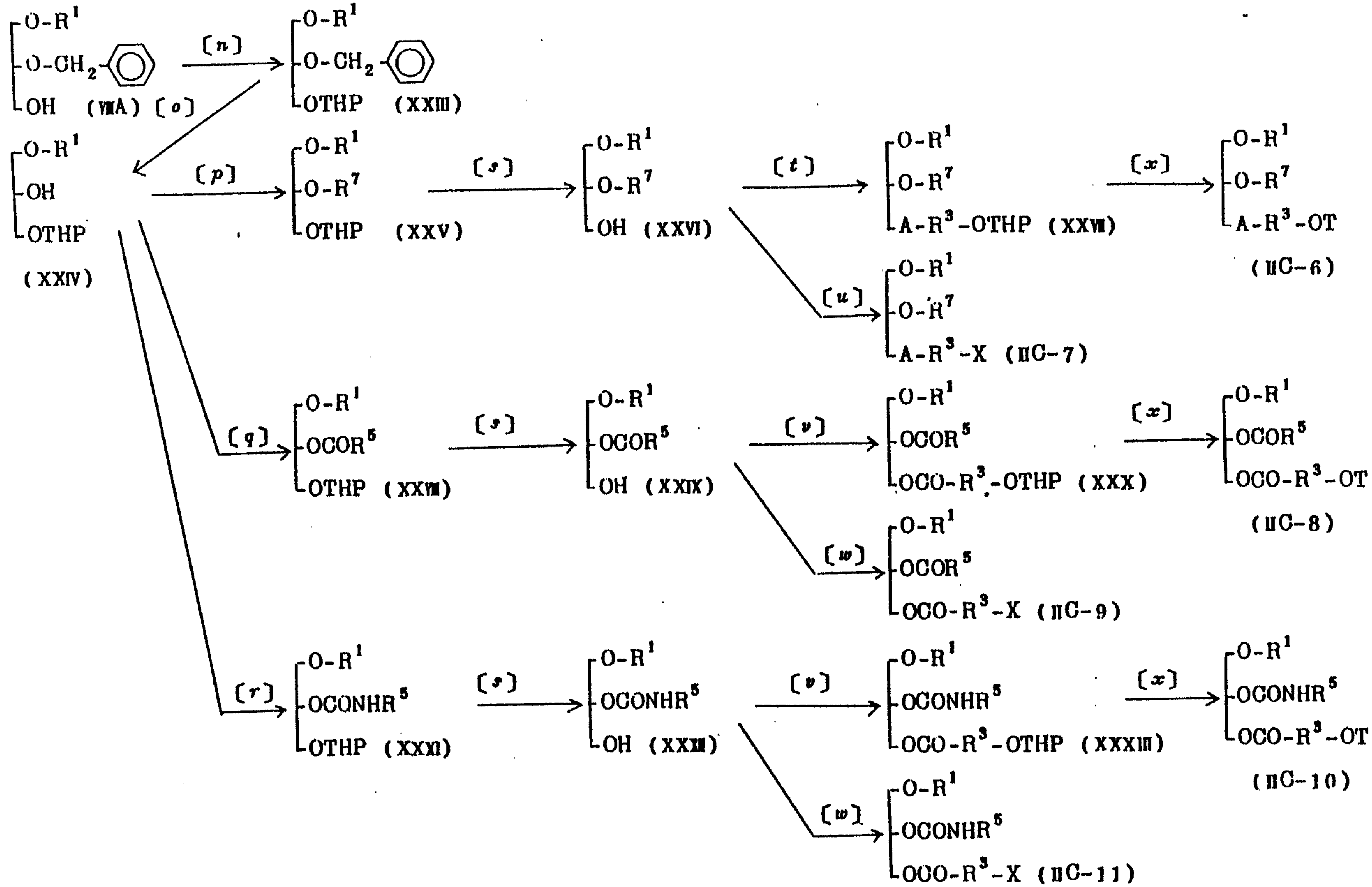


(VIIA) (XXIII) (XXIV) (XXV) (XXVI) (XXVII) (IIC-6) (IIC-7) (XXVIII) (XXIX) (XXX) (IIC-8) (IIC-9) (XXXI) (XXXII) (XXXIII) (IIC-10) (IIC-11)

Scheme III (continued)

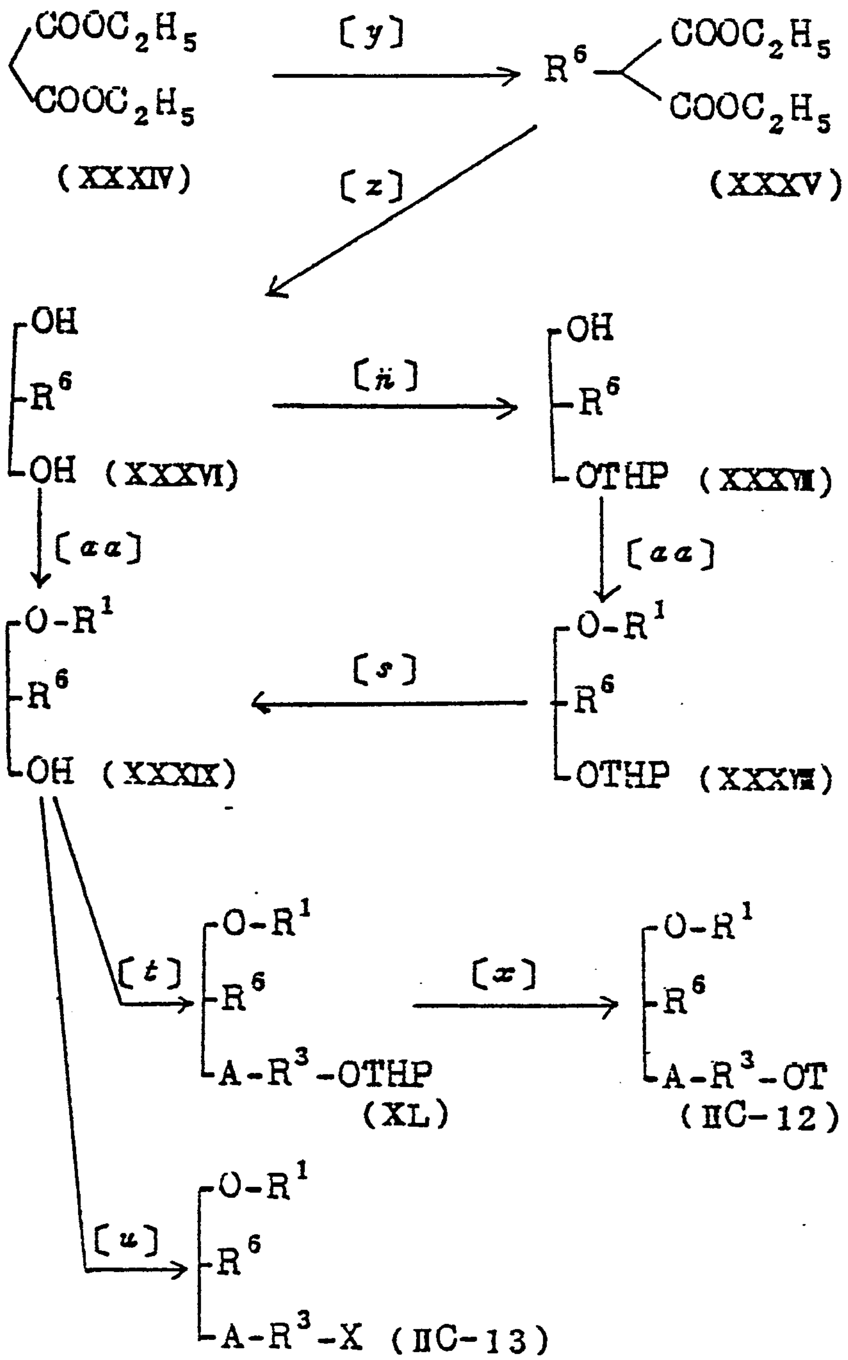


(XXXIV) (XXXV) (XXXVI) (XXXVII) (XXXIX) (XXXVIII) (XL) (IIC-12) (IIC-13)

In Scheme II and III, each step can be effected using methods known *per se*. For example, step (i) may be carried out by methods hereinbefore described for step (e).

Step (j) may be carried out by methods hereinbefore described for step (a) by using a compound of the general formula:

$$Q\text{-}R^7 \qquad \text{(XLI)}$$

(wherein the various symbols are as hereinbefore defined) instead of a compound of the general formula (XIV).

Step (k) may be carried out by methods hereinbefore described for the esterification (4).

Step (l) may be carried out by methods hereinbefore described for the reaction (5).

Step (m) may be carried out by methods hereinbefore described for step (b), and then for step (c).

Step (n) may be carried out by using 2,3-dihydropyran in methylene chloride in the presence of a small amount of p-toluenesulfonic acid at room temperature.

Step (o) may be carried out by methods hereinbefore described for step (e).

Step (p), (q) and (r) may be carried out by methods hereinbefore described for step (j), (k) and (l), respectively.

Step (s) may be carried out by methods hereinbefore described for step (b).

Step (t) may be carried out by methods hereinbefore described for step (a) or step (g), and step (u) may be carried out by methods hereinbefore described for step (f) or step (h).

Step (v) and (w) may be carried out by methods hereinbefore described for step (g) and (h), respectively.

Step (x) may be carried out by methods hereinbefore described for step (b), and then for step (c).

Step (y) may be carried out by reacting diethyl malonate of the formula (XXXIV) with a compound of the general formula:

$$X^1-R^6 \qquad \text{(XLII)}$$

(wherein $X^1$ represents a halogen atom and $R^6$ is as hereinbefore defined) in the presence of an alkoxide of alkali metal prepared by using an alkali metal such as sodium in a lower alkanol such as methanol or ethanol, at a temperature from ambient to the reflux temperature of the reaction mixture.

Step (z) may be carried out by using a reductant such as lithium aluminium hydride in an inert organic solvent such as diethyl ether or tetrahydrofuran at a temperature from ambient to the relfux temperature of the reaction mixture.

Step (aa) may be carried out by methods hereinbefore described for step (a) by using a compound of the general formula:

Q-$R^1$ (XLIII)

(wherein the various symbols are as hereinbefore defined) instead of a compound of the general formula (XIV).

In compounds of the general formula (II) used as starting materials, all of compounds wherein $R^2$ represents an azido group, an amino group or a group of the formula: $-NHCOR^5$, $-NHCOOR^5$ or $-NHCONHR^5$, in which $R^5$ is as hereinbefore defined, may be prepared by methods described in any one of the reference "A".

In compounds of the general formula (IV) used as starting materials, compounds wherein $R^2$ represents an alkyl group of 1 to 20 carbon atoms or an alkenyl group of 2 to 20 carbon atoms, compounds wherein $R^2$ represents an alkoxy group of 1 to 20 carbon atoms or an alkenyloxy group of 3 to 20 carbon atoms, and compounds wherein $R^2$ represents a group of the formula: $-O-CH_2-$ 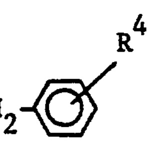 , in which $R^4$ is as hereinbefore defined, are represented as compounds of the general formula (XXXIX), (XXVI) and (VIII), respectively.

In compounds of the general formula (IV) used as starting materials, all of compounds wherein $R^2$ represents an azido group or a group of the formula: $-NHCOR^5$, $-NHCOOR^5$ or $-NHCONHR^5$, in which $R^5$ is as hereinbefore defined, may be prepared by methods described in any one of the reference "A".

According to a further feature of the present invention, in the glycerol derivatives of the general formula (I), compounds of the general formula:

 

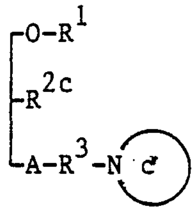

(IC)

(wherein (i) when A is an oxygen atom, $R^{2c}$ represents a hydroxy group, an amino group, an azido group, or a group of the formula: $-OCOR^5$, $-OCONHR^5$, $-NHCOR^5$, $-NHCOOR^5$ or $-NHCONHR^5$, in which $R^5$ is as hereinbefore defined, or (ii) when A is a carbonyloxy group, $R^{2c}$ represents a hydroxy group, or a group of the formula: $-OCOR^5$ or $-OCONHR^5$, in which $R^5$ is as hereinbefore defined, 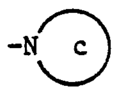 is as hereinbefore defined for 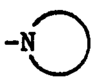, with the proviso that the group should not be substituted by hydroxy group(s) regardless of the definition of $R^{2c}$, and the other symbols are as hereinbefore defined) may also be prepared by the series of reactions depicted schematically below in Scheme IV.

Scheme IV

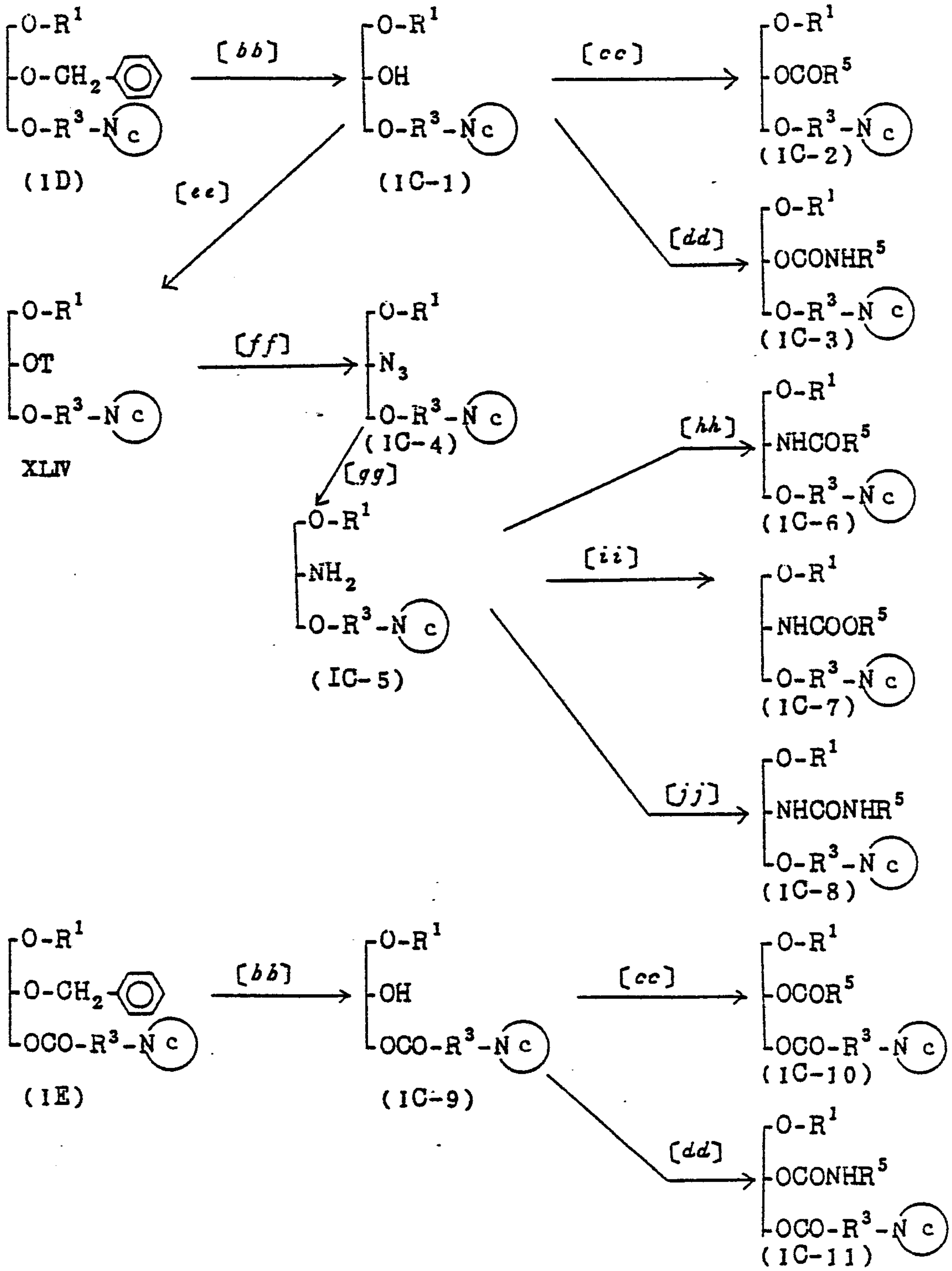


(1D) (IC-1) (IC-2) (IC-3) XLIV (IC-4) (IC-5) (IC-6) (IC-7) (IC-8) (1E) (IC-9) (IC-10) (IC-11)

In Scheme IV, each step can be effected using methods known *per se*. For example, step (bb) may be carried out by methods hereinbefore described for step (e).

Step (cc) and (dd) may be carried out by methods hereinbefore described for step (k) and (l), respectively.

Step (ee) may be carried out by methods hereinbefore described for step (c).

Step (ff) may be carried out by using sodium azide in an inert organic solvent such as hexamethylphosphoramide, dimethylformamide or dimethyl sulfoxide at a temperature from ambient to 80°C.

Step (gg) is the reduction, and may be carried out by methods hereinbefore described for step (e), or when the group 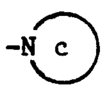 is a tertiary cyclic amine, by using lithium aluminium hydride in a suitable inert organic solvent, e.g. tetrahydrofuran or diethyl ether at a temperature from 0°C to the reflux temperature of the reaction mixture.

Step (hh) may be carried out by reacting an amine compound of the general formula (IC-5) with an acyl halide of the general formula:

$$X^2\text{-CO-}R^5 \qquad \text{(XLV)}$$

(wherein $X^2$ represents a halogen atom and $R^5$ is as hereinbefore defined) or with an acid anhydride of the general formula:

$$R^5\text{-CO-O-CO-}R^5 \qquad \text{(XLVI)}$$

(wherein $R^5$ is as hereinbefore defined) in an inert organic solvent, e.g. ethyl acetate, benzene, hexane, diethyl ether, tetrahydrofuran, chloroform, methylene chloride or carbon tetrachloride, or a mixture of two or more of them, or in the absence of a solvent, in the presence of a tertiary amine such as pyridine or triethylamine at a temperature from 0°C to 50°C.

Step (ii) may be carried out by methods hereinbefore described for step (hh) by using an ester of haloformic acid of the general formula:

$$X^3\text{-COOR}^5 \qquad \text{(XLVII)}$$

(wherein $X^3$ represents a halogen atom and $R^5$ is as hereinbefore defined) instead of a compound of the general formula (XLV) or (XLVI).

Step (jj) may be carried out by methods hereinbefore described for step (hh) by using an isocyanate of the general formula (VII) instead of a compound of the general formula (XLV) or (XLVI).

Compounds of the general formula (XIV) (XV), (XVI), (XVII), (XVIII), (XLI), (XLII), (XLIII), (XLV), (XLVI) and (XLVII) used in the reactions hereinbefore described, are known *per se* or may be easily prepared by methods known *per se*.

Compounds of the general formula (VIII) used as starting materials may be prepared by methods described in any one of the reference "A". Diethyl malonate of the formula (XXXIV) used as starting material is known per se.

Furthermore, compounds of the general formula (XXVI) wherein $R^7$ represents an alkyl group of 1 to 20 carbon atoms in Scheme III (hereinafter depicted in the general formula (XXVIA)), compounds of the general formula (XXVII) wherein $R^7$ represents an alkyl group of 1 to 20 carbon atoms and A represents an oxygen atom in Scheme III ( hereinafter depicted in the general formula (XXVIIA)) and compounds of the general formula (IC-1) in Scheme IV, may also be prepared by the series of reactions depicted schematically below in Scheme V, wherein $R^{7a}$ represents an alkyl group of 1 to 20 carbon atoms and the other symbols are as hereinbefore defined.

Scheme V

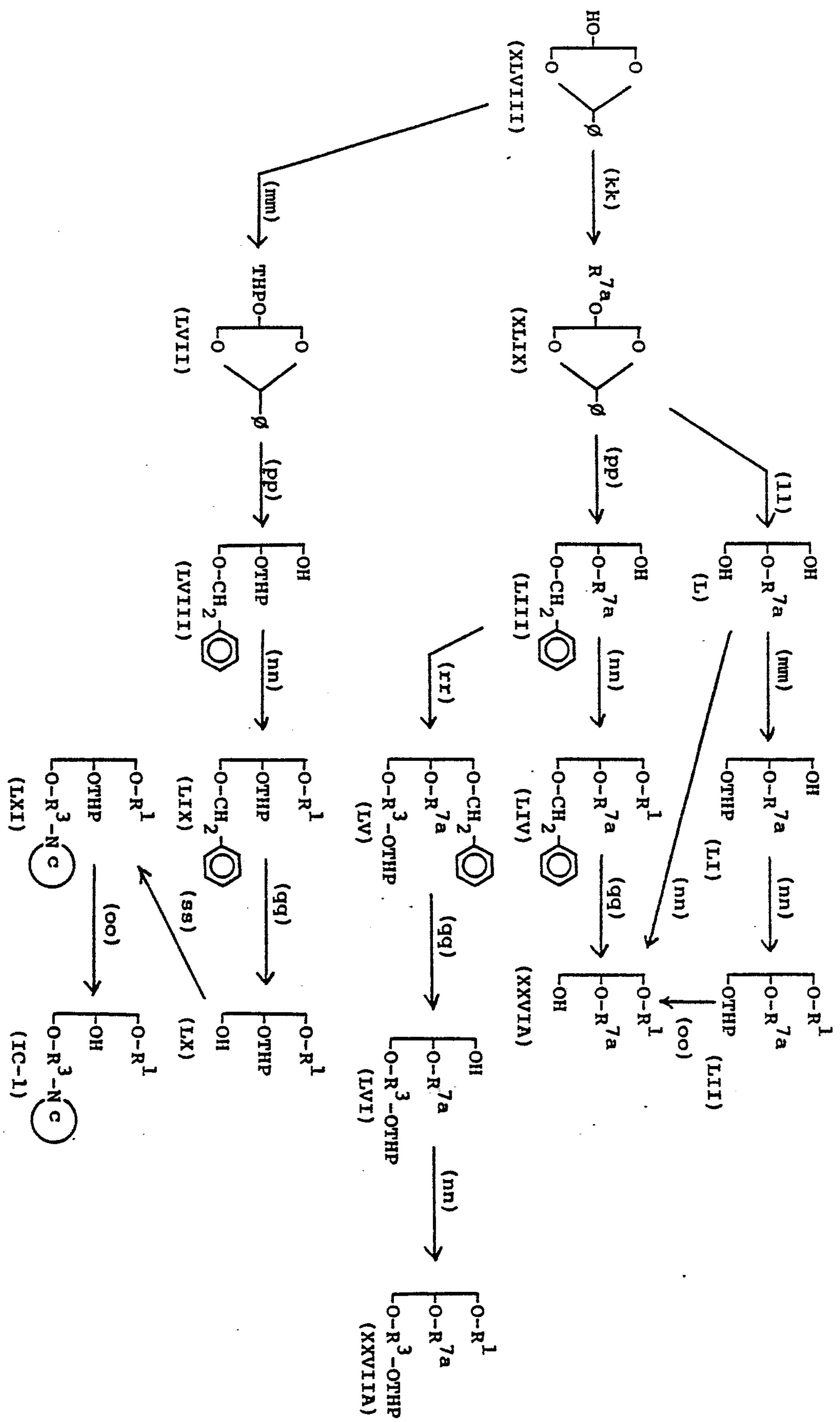
(XLVIII)
(kk)
(mm)
(XLIX)
(LVII)
(ll)
(pp)
(pp)
(L)
(LIII)
(LVIII)
(mm)
(nn)
(rr)
(nn)
(LI)
(LIV)
(LV)
(LIX)
(LXI)
(nn)
(nn)
(qq)
(qq)
(qq)
(ss)
(oo)
(LII)
(XXVIA)
(oo)
(LVI)
(LX)
(IC-1)
(nn)
(XXVIIA)

In Scheme V, each step can be effected using methods known per se. For example, step (kk) may be carried out by methods hereinbefore described for step (a) by using a compound of the general formula:

$$Q\text{-}R^{7a} \qquad \text{(LXII)}$$

(wherein the various symbols are as hereinbefore defined) instead of a compound of the general formula (XIV).

Step (ll) may be carried out by methods hereinbefore described for step (b).

Step (mm) may be carried out by methods hereinbefore described for step (n).

Step (nn) may be carried out by methods hereinbefore described for step (aa).

Step (oo) may be carried out by methods hereinbefore described for step (b).

Step (pp) may be carried out by using diisobutylaluminium hydride in toluene or lithium aluminium hydride in the presence of aluminium chloride in a suitable inert organic solvent, e.g. a halogenated hydrocarbon such as methylene chloride, chloroform or carbon tetrachloride, hydrocarbon such as benzene or toluene or diethyl ether, or a mixture of two or more of them, at a temperature from ambient to the reflux temperature of the reaction mixture.

Step (qq) may be carried out by methods hereinbefore described for step (e).

Step (rr) may be carried out by methods hereinbefore described for step (a).

Step (ss) may be carried out by methods hereinbefore described for the conversion of compounds of the general formula (VIII) to those of the general formula (IA) or (IB-1), wherein A represents an oxygen atom and $R^2$ represents a group of the formula: $-O-CH_2-$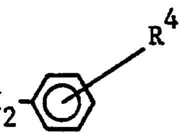, via a compound of the general formula (IIB-1) or (IIB-3).

Glycerol 1,3-O-benzylidene ether of the formula (XLVIII), used as starting material is a known compound described in J. Amer. Chem, Soc., 50, 2235 (1928).

Glycerol derivatives of the general formula (I) wherein 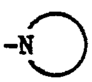 represents a tertiary cyclic amine or other secondary or tertiary nitrogen atom exists in the ring of 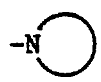 (hereinafter depicted in the general formula (IF)) may be converted into acid addition salts thereof. Preferably, acid addition salts are non-toxic salts and are water-soluble. Suitable acid addition salts are, for example, inorganic acid salts such as hydrochloride, hydrobromide, hydroiodide, sulfate, phosphonate, nitrate, or organic acid salts such as acetate, lactate, tartrate, benzoate, citrate, methanesulphonate, ethanesulphonate, benzenesulphonate, toluenesulphonate or isethionate. Acid addition salts may be prepared by methods known per se, for example, reacting stoichiometric quantities of a compound of the general formula (IF) and a desirable acid in a suitable solvent.

Glycerol derivatives of the general formula (I) and acid addition salts thereof have antagonistic effect on PAF, hypotensive effect like PAF, inhibitory effect on phospholipase and further, inhibitory effect on growth of tumour cell or induction of differentiation, and are, therefore, useful as a platelet aggregation inhibitor, anti-asthmatic agent, anti-allergic agent, anti-inflammatory agent, hypotensive agent and anti-tumour agent for mammals including human beings, especially human beings. These effect were confirmed by standard laboratory test and, for example,antagonistic effect on PAF was confirmed by the following screening system.

Inhibitory effect on PAF-induced platelet aggregation

Whole blood of male guinea-pig was collected with a 3.8% aqueous solution of trisodium citrate in a proportion of 9:1 (v/v) and the mixture was centrifuged for 10 minutes at room temperature (120 x g) to obtain platelet rich plasma. Using obtained plasma, PAF (10nM)-induced aggregation was determined by the absorbancy method using an aggregometer of Born [cf. J. Physiol., 162, 67 (1962)].

The evaluation is conducted using the concentration of compounds of the present invention required to produce a 50% inhibition of the effect, i.e. $IC_{50}$. The results are shown in the following table.

Table

| Example No. of tested compounds | Inhibitory effect on PAF-induced platelet aggregation ($IC_{50}$,M) |
|---|---|
| 1(b) | $3.5 \times 10^{-6}$ |
| 1(i) | $6.2 \times 10^{-7}$ |
| 1(l) | $3.8 \times 10^{-7}$ |
| 1(o) | $6.0 \times 10^{-6}$ |
| 2(c) | $7.7 \times 10^{-6}$ |
| 11(a) | $1.8 \times 10^{-6}$ |
| 11(c) | $1.9 \times 10^{-6}$ |

On the other hand, it was confirmed that the acute toxicity of all extent of the compounds of the present invention was more than 50 mg/kg by intravenous administration. Therefore, glycerol derivatives of the present invention may be considered to be sufficiently safe and suitable for medical use. For example, the mortality (dead/used) in glycerol derivatives of the present invention, i.e. compounds prepared in Example 1(h) and 11(a) was 0/5 and 0/5, respectively, when each glycerol compound was intravenously administered to tail vein in five mice at the dose of 50 mg/kg.

For the purpose hereinbefore described, glycerol

derivatives of the general formula (I), or non-toxic acid addition salts thereof may normally be administered systemically or partially, usually by oral or parenteral (e.g. intravenous, subcutaneous or intramuscular) administration. The dose to be administered is determined depending upon, for example, age, body weight, symptoms, the desired therapeutic effect, the route of administration, and the duration of the treatment etc.

In the human adult, the doses per person are generally between 1 mg and 1 g, preferebly between 20 mg and 200 mg by oral administration up to several times per day, and between 100 µg and 100 mg, preferably between 1 mg and 10 mg by parenteral administration up to several times per day.

As mentioned above, the doses to be used depend on various conditions. Therefore, there are cases in which doses lower than the ranges specified above and doses greater than the ranges specified above, may be used.

The present invention includes within its scope pharmaceutical compositions which comprise at least one glycerol derivative of the general formula (I), or non-toxic acid addition salts thereof, together with a pharmaceutical carrier or coating.

Solid compositions according to the present invention for oral administration include compressed tablets, pills, dispersible powders and granules. In such solid compositions, one or more of the active compound(s) is, or are, admixed with at least one inert diluent such as lactose, mannitol, glucose, hydroxypropyl cellulose, microcrystalline cellulose, starch, polyvinyl-pyrrolidone or magnesium metasilicate aluminate. The

compositions may also comprise, as is normal practice, additional substances other than inert diluents e.g. lubricating agents such as magnesium stearate, and, disintegrating agents such as cellulose calcium gluconate. The tablets or pills may, if desired, be made into gastric film-coated or enteric film-coated tablets or pills, such as sugar-coated, gelatin-coated, hydroxypropylcellulose-coated or hydroxypropylmethylcellulose phthalate-coated tablets or pills; two or more layers may be used. The compositions for oral administration also include capsules of absorbable material such as gelatin.

Liquid compositions for oral administration include pharmaceutically-acceptable emulsions, solutions, suspensions, syrups and elixirs containing inert diluents commonly used in the art such as distilled water or ethanol. Besides inert diluents such compositions may also comprise adjuvants such as wetting and suspending agents, and sweetening, flavouring, perfuming and preserving agents.

Other compositions for oral administration include spray compositions which may be prepared by known methods and which comprise one or more of the active compound(s).

Preparations for injection according to the present invention for parenteral administration include sterile aqueous or non-aqueous solutions, suspensions or emulsions. Examples of aqueous solvents or suspending media are distilled water for injection and physiological salt solution. Examples of non-aqueous solvents or suspending media are propylene glycol, polyethylene glycol, vegetable oils such as olive oil, alcohols such as ethanol, Polysorbate 80 (registered Trade Mark). These

compositions may also include adjuvants such as preserving, wetting, emulsifying and dispersing agents. They may be sterilized, for example, by filtration through a bacteria-retaining filter, by incorporation of sterilizing agents in the compositions or by irradiation. They may also be manufactured in the form of sterile solid compositions which can be dissolved in sterile water or some other sterile injectable medium immediately before use.

Other compositions for parenteral administration include liquids for external use, and endermic liniments such as ointments, suppositories for rectal administration and pessaries for vaginal administration which comprise one or more of the active compound(s) and may be prepared by known methods.

The percentage of active ingredient in the compositions of the invention may be varied, it being necessary that it should constitute a proportion such that a suitable dosage for the therapeutic effect desired shall be obtained. Obviously several unit desage forms may be administered at about the same time. In general, the preparations should normally contain at least 0.025% by weight of active substance when required for administration by injection; for oral administration the preparations will normally contain at least 0.1% by weight of active substance.

Preferred compounds of the general formula (I) of the present invention are, for example, as follows:

(2RS)-1-O-hexadecyl-3-O-[7-(N-methyl-1-pyrrolidinio)heptyl]-glycerol halide or its mesylate,

(2RS)-1-O-hexadecyl-3-O-[7-(3-thiazolio)heptyl]glycerol halide or

its mesylate,

(2RS)-1-O-hexadecyl-2-dehydroxy-2-amino-3-O-[7-(N-methyl-1-pyrrolidinio)heptyl] glycerol halide or its mesylate,

(2RS)-1-O-hexadecyl-2-dehydroxy-2-amino-3-O-[7-(3-thiazolio)-heptyl]glycerol halide or its mesylate,

(2RS)-1-O-hexadecyl-2-dehydroxy-2-azido-3-O-[7-(N-methyl-1-pyrrolidinio)heptyl]glycerol halide or its mesylate,

(2RS)-1-O-hexadecyl-2-dehydroxy-2-azido-3-O-[7-(3-thiazolio)-heptyl]glycerol halide or its mesylate,

(2RS)-1-O-hexadecyl-2-dehydroxy-2-ethyl-3-O-[7-(N-methyl-1-pyrrolidinio)heptyl]glycerol halide or its mesylate,

(2RS)-1-O-hexadecyl-2-dehydroxy-2-ethyl-3-O-[7-(3-thiazolio)-heptyl]glycerol halide or its mesylate,

(2RS)-1-O-hexadecyl-2-dehydroxy-2-propyl-3-O-[7-(N-methyl-1-pyrrolidinio)heptyl]glycerol halide or its mesylate,

(2RS)-1-O-hexadecyl-2-dehydroxy-2-propyl-3-O-[7-(3-thiazolio)-heptyl]glycerol halide or its mesylate,

(2RS)-1-O-hexadecyl-2-dehydroxy-2-allyl-3-O-[7-(N-methyl-1-pyrrolidinio)heptyl]glycerol halide or its mesylate,

(2RS)-1-O-hexadecyl-2-dehydroxy-2-allyl-3-O-[7-(3-thiazolio)-heptyl]glycerol halide or its mesylate,

(2RS)-1-O-hexadecyl-2-O-allyl-3-O-[7-(N-methyl-1-pyrrolidinio)-heptyl]glycerol halide or its mesylate,

(2RS)-1-O-hexadecyl-2-O-allyl-3-O-[7-(3-thiazolio)heptyl glycerol halide]or its mesylate,

(2RS)-1-O-hexadecyl-2-O-acetyl-3-O-[7-(N-methyl-1-pyrrolidinio)-

heptyl]glycerol halide or its mesylate,
(2RS)-1-O-hexadecyl-2-O-acetyl-3-O-[7-(3-thiazolio)heptyl]-glycerol halide or its mesylate,
(2RS)-1-O-hexadecyl-2-O-(N-methylcarbamoyl)-3-O-[7-(N-methyl-1-pyrrolidinio)heptyl]glycerol halide or its mesylate,
(2RS)-1-O-hexadecyl-2-O-(N-methylcarbamoyl)-3-O-[7-(3-thiazolio)-heptyl]glycerol halide or its mesylate,
(2RS)-1-O-hexadecyl-2-dehydroxy-2-acetylamino-3-O-[7-(N-methyl-1-pyrrolidinio)heptyl]glycerol halide or its mesylate,
(2RS)-1-O-hexadecyl-2-dehydroxy-2-acetylamino-3-O-[7-(3-thiazolio)heptyl]glycerol halide or its mesylate,
(2RS)-1-O-hexadecyl-2-dehydroxy-2-(N'-methylureido)-3-O-[7-(N-methyl-1-pyrrolidinio)heptyl]glycerol halide or its mesylate and
(2RS)-1-O-hexadecyl-2-dehydroxy-2-(N'-methylureido)-3-O-[7-(3-thiazolio)heptyl]glycerol halide or its mesylate, and the corresponding (N-methyl-piperidinio) compounds, (N-methyl-4-hydroxypiperidinio) compounds, (N-methyl-2,6-dimethylpiperidinio) compounds, (N-methyl-hexamethyleneiminio) compounds, (N,N',N'-trimethyl-1-piperazinio) compounds, (1-methyl-3-imidazolio) compounds, (1-pyridinio) compounds, (1-quinuclidinio) compounds, (triethylenediamin-1-io) compounds and (N-methylmorpholinio) compounds, and the corresponding 4-substituted butyl compounds, 5-substituted pentyl compounds, 6-substituted hexyl compounds and 8-substituted octyl comounds (i.e. compounds of the general formula (I) wherein $R^3$ is tetramethylene, pentamethylene,

hexamethylene and octamethylene group, respectively).

In addition, other preferred compounds are as follows:

(2RS)-1-O-hexadecyl-2-O-ethyl-3-O-[7-(N-methyl-1-pyrrolidinio)-heptyl]glycerol halide or its mesylate,

(2RS)-1-O-hexadecyl-2-O-ethyl-3-O-[7-(3-thiazolio)heptyl]glycerol halide or its mesylate,

(2RS)-1-O-hexadecyl-2-O-ethyl-3-O-[7-(N-methyl-piperidinio)heptyl]glycerol halide or its mesylate,

(2RS)-1-O-hexadecyl-2-O-ethyl-3-O-[7-(N-methyl-4-hydroxy-piperidinio)heptyl]glycerol halide or its mesylate,

(2RS)-1-O-hexadecyl-2-O-ethyl-3-O-[7-(N-methyl-2,6-dimethyl-piperidinio)heptyl]glycerol halide or its mesylate,

(2RS)-1-O-hexadecyl-2-O-ethyl-3-O-[7-(N-methyl-hexamethylene-iminio)heptyl]glycerol halide or its mesylate,

(2RS)-1-O-hexadecyl-2-O-ethyl-3-O-[7-(N,N',N'-trimethyl-1-piperazinio)heptyl]glycerol halide or its mesylate,

(2RS)-1-O-hexadecyl-2-O-ethyl-3-O-[7-(1-methyl-3-imidazolio)-heptyl]glycerol halide or its mesylate,

(2RS)-1-O-hexadecyl-2-O-ethyl-3-O-[7-(1-pyridinio)-heptyl]glycerol halide or its mesylate,

(2RS)-1-O-hexadecyl-2-O-ethyl-3-O-[7-(1-quinuclidinio)-heptyl]glycerol halide or its mesylate,

(2RS)-1-O-hexadecyl-2-O-ethyl-3-O-[7-(triethylenediamin-1-io)-heptyl]glycerol halide or its mesylate,

(2RS)-1-O-hexadecyl-2-O-ethyl-3-O-[7-(N-methylmorpholinio)-heptyl]glycerol halide or its mesylate,

(2RS)-1-O-hexadecyl-2-O-propyl-3-O-[7-(N-methyl-1-pyrrolidinio)-heptyl]glycerol halide or its mesylate,

(2RS)-1-O-hexadecyl-2-O-propyl-3-O-[7-(3-thiazolio)heptyl]-glycerol halide or its mesylate,

(2RS)-1-O-hexadecyl-2-O-propyl-3-O-[7-(N-methyl-1-piperidinio)heptyl]glycerol halide or its mesylate,

(2RS)-1-O-hexadecyl-2-O-propyl-3-O-[7-(N-methyl-4-hydroxy-piperidinio)heptyl]glycerol halide or its mesylate,

(2RS)-1-O-hexadecyl-2-O-propyl-3-O-[7-(N-methyl-2,6-dimethyl-piperidinio)heptyl]glycerol halide or its mesylate,

(2RS)-1-O-hexadecyl-2-O-propyl-3-O-[7-(N-methyl-hexamethylene-iminio)heptyl]glycerol halide or its mesylate,

(2RS)-1-O-hexadecyl-2-O-propyl-3-O-[7-(N,N',N'-trimethyl-1-piperazinio)heptyl]glycerol halide or its mesylate,

(2RS)-1-O-hexadecyl-2-O-propyl-3-O-[7-(1-methyl-3-imidazolio)-heptyl]glycerol halide or its mesylate,

(2RS)-1-O-hexadecyl-2-O-propyl-3-O-[7-(1-pyridinio)-heptyl]glycerol halide or its mesylate,

(2RS)-1-O-hexadecyl-2-O-propyl-3-O-[7-(1-quinuclidinio)-heptyl]glycerol halide or its mesylate,

(2RS)-1-O-hexadecyl-2-O-propyl-3-O-[7-(triethylenediamin-1-io)-heptyl]glycerol halide or its mesylate,

(2RS)-1-O-hexadecyl-2-O-propyl-3-O-[7-(N-methylmorpholinio)-heptyl]glycerol halide or its mesylate,

(2RS)-1-O-hexadecyl-2-O-benzyl-3-O-[7-(N-methyl-1-pyrrolidinio)-heptyl]glycerol halide or its mesylate,

(2RS)-1-O-hexadecyl-2-O-benzyl-3-O-[7-(3-thiazolio)heptyl]-glycerol halide or its mesylate,

(2RS)-1-O-hexadecyl-2-O-benzyl-3-O-[7-(N-methyl-1-piperidinio)-heptyl]glycerol halide or its mesylate,

(2RS)-1-O-hexadecyl-2-O-benzyl-3-O-[7-(N-methyl-4-hydroxy-piperidinio)heptyl]glycerol halide or its mesylate,

(2RS)-1-O-hexadecyl-2-O-benzyl-3-O-[7-(N-methyl-2,6-dimethyl-piperidinio)heptyl]glycerol halide or its mesylate,

(2RS)-1-O-hexadecyl-2-O-benzyl-3-O-[7-(N-methyl-hexamethylene-iminio)heptyl]glycerol halide or its mesylate,

(2RS)-1-O-hexadecyl-2-O-benzyl-3-O-[7-(N,N',N'-trimethyl-1-piperazinio)heptyl]glycerol halide or its mesylate,

(2RS)-1-O-hexadecyl-2-O-benzyl-3-O-[7-(1-methyl-3-imidazolio)-heptyl]glycerol halide or its mesylate,

(2RS)-1-O-hexadecyl-2-O-benzyl-3-O-[7-(1-pyridinio)-heptyl]glycerol halide or its mesylate,

(2RS)-1-O-hexadecyl-2-O-benzyl-3-O-[7-(1-quinuclidinio)-heptyl]glycerol halide or its mesylate,

(2RS)-1-O-hexadecyl-2-O-benzyl-3-O-[7-(triethylenediamin-1-io)-heptyl]glycerol halide or its mesylate,

(2RS)-1-O-hexadecyl-2-O-benzyl-3-O-[7-(N-methylmorpholinio)-heptyl]glycerol halide or its mesylate,

(2RS)-1-O-hexadecyl-2-dehydroxy-2-isobutoxycarbonylamino-3-O-[7-(N-methyl-1-pyrrolidinio)heptyl]glycerol halide or its mesylate,

(2RS)-1-O-hexadecyl-2-dehydroxy-2-isobutoxycarbonylamino-3-O-[7-(3-thiazolio)heptyl]glycerol halide or its mesylate,

(2RS)-1-O-hexadecyl-2-dehydroxy-2-isobutoxycarbonylamino-3-O-[7-(N-methyl-1-piperidinio)heptyl]glycerol halide or its mesylate,

(2RS)-1-O-hexadecyl-2-dehydroxy-2-isobutoxycarbonylamino-3-O-[7-(N-methyl-4-hydroxypiperidinio)heptyl]glycerol halide or its

mesylate,

(2RS)-1-O-hexadecyl-2-dehydroxy-2-isobutoxycarbonylamino-3-O-[7-(N-methyl-2,6-dimethylpiperidinio)heptyl]glycerol halide or its mesylate,

(2RS)-1-O-hexadecyl-2-dehydroxy-2-isobutoxycarbonylamino-3-O-[7-(N-methyl-hexamethyleneiminio)heptyl]glycerol halide or its mesylate,

(2RS)-1-O-hexadecyl-2-dehydroxy-2-isobutoxycarbonylamino-3-O-[7-(N,N',N'-trimethyl-1-piperazinio)heptyl]glycerol halide or its mesylate,

(2RS)-1-O-hexadecyl-2-dehydroxy-2-isobutoxycarbonylamino-3-O-[7-(1-methyl-3-imidazolio)heptyl]glycerol halide or its mesylate,

(2RS)-1-O-hexadecyl-2-dehydroxy-2-isobutoxycarbonylamino-3-O-[7-(1-pyridinio)heptyl]glycerol halide or its mesylate,

(2RS)-1-O-hexadecyl-2-dehydroxy-2-isobutoxycarbonylamino-3-O-[7-(1-quinuclidinio)heptyl]glycerol halide or its mesylate,

(2RS)-1-O-hexadecyl-2-dehydroxy-2-isobutoxycarbonylamino-3-O-[7-(triethylenediamin-1-io)heptyl]glycerol halide or its mesylate and

(2RS)-1-O-hexadecyl-2-dehydroxy-2-isobutoxycarbonylamino-3-O-[7-(N-methylmorpholinio)heptyl]glycerol halide or its mesylate, and the corresponding 4-substituted butyl compounds, 5-substituted pentyl compounds, 6-substituted hexyl compounds and 8-substituted octyl comounds (i.e. compounds of the general formula (I) wherein $R^3$ is tetramethylene, pentamethylene, hexamethylene and octamethylene group, respectively), and the corresponding ester compounds (i.e. comounds of the general formula (I) wherein A is a carbonyloxy group).

The following Reference Examples and Examples illustrate, but not limit, the preparation of compounds of the present invention. In the Reference Examples and Examples, "TLC", "NMR", "IR" and "MS" represent "Thin layer chromatography", "Nuclear magnetic resonance spectrum", "Infrared absorption spectrum" and "Mass spectrum", respectively. The solvents in parenthesis specified in chromatographic separations show the developing solvents used. Except when specified otherwise, infrared absorption spectra were recorded by the liquid film method and nuclear magnetic resonance spectra were recorded in deuterochloroform ($CDCl_3$) solution. In structural formulae, "Ø", "Ms" and "THP" represent "phenyl group", "methanesulfonyl (mesyl) group" and "tetrahydropyran-2-yl group", respectively.

Reference Example 1

Synthesis of 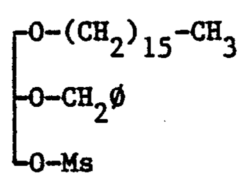

To a mixture of one g of (2RS)-1-O-hexadecyl-2-O-benzylglycerol (the compound described in the specification of the European Patent Publication No. 109255, Reference Example 5 ), 10 ml of methylene chloride and one ml of

triethylamine, was added 0.24 ml of mesyl chloride under cooling with ice, and the mixture was stirred for 30 minutes. To the reaction mixture was added 100 ml of ethyl acetate and the obtained solution was washed with water and a saturated aqueous solution of sodium chloride, successively, dried over anhydrous magnesium sulfate and concentrated under reduced pressure to give 1.2 g of the crude title compound having the following physical data:

TLC(chloroform:acetone=10:1): Rf= 0.86.

Reference Example 2

Synthesis of

$$\begin{cases} -O-(CH_2)_{15}-CH_3 \\ -O-CH_2\phi \\ -O-(CH_2)_4-OH \end{cases}$$

Under an atmosphere of nitrogen, to a suspension of 2.5 g of sodium hydride (content: 64.1%) in 200 ml of dry dimethylformamide was added 4 g of 1,4-butanediol and the mixture was stirred for 10 minutes at room temperature. To the obtained solution was added a solution of 14.5 g of the mesyl compound (prepared in Reference Example 1) in 100 ml of dry dimethylformamide and the obtained solution was stirred for 2 hours at 70°C. To the reaction mixture was added 4 ml of water and the mixture was concentrated under reduced pressure. To the obtained residue was added 500 ml of ethyl acetate and the solution was washed with water and a saturated aqueous solution

of sodium chloride, successively, dried over anhydrous sodium sulfate and concentrated under reduced pressure. The residue was purified by column chromatography on silica gel (eluent, ethyl acetate:n-hexane=1:3) to give 8.4 g of the title compound having the following physical data:

TLC(chloroform:acetone=10:1): Rf= 0.43;

NMR: δ= 7.2 (5H, bs), 4.6 (2H, s), 3.9-3.2 (11H, m), 2.75 (1H, bs), 2.0-0.8 (35H, m);

IR: ν= 3400, 2930, 2850, 1460, 1110 $cm^{-1}$;

MS: m/e= 478 ($M^+$), 406, 372, 297, 282.

By the same procedure as described in Reference Example 2, the following compounds were prepared.

(a)

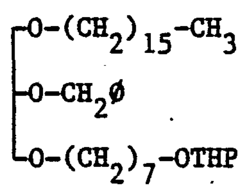

Starting materials: (2RS)-1-O-hexadecyl-2-O-benzylglycerol and 7-(tetrahydropyran-2-yloxy)-1-tosyloxyheptane;

TLC(ethyl acetate:n-hexane=1:5): Rf= 0.55;

NMR: δ= 7.2 (5H, s), 4.63 (2H, s), 4.6-4.3 (1H, m), 4.0-3.0 (13H, m), 2.0-0.6 (47H, m) ;

MS : m/e = 604 ($M^+$), 519.

(b)

$-O-(CH_2)_{15}-CH_3$
$-O-CH_2Ø$
$-O-(CH_2)_4-Cl$

Starting materials: (2RS)-1-O-hexadecyl-2-O-benzylglycerol and 4-tosyloxybutyl chloride;

TLC(ethyl acetate:n-hexane=1:4): Rf= 0.69;

NMR : $\delta$ = 7.18 (5H, s), 4.62 (2H, s), 3.8 - 3.13 (11H, m), 2.13 - 0.63 (37H, m);

IR : $\nu$ = 2920, 2850, 1460, 1450, 1115, 735, 695 ;

MS : m/e = 496 ($M^+$), 434, 405, 390, 214.

Reference Example 3

Synthesis of

$-O-(CH_2)_{15}-CH_3$
$-O-CH_2Ø$
$-O-(CH_2)_4-OTHP$

A mixture of 8 g of the hydroxy comound (prepared in Reference Example 2), 1.5 g of 2,3-dihydropyran, 20 mg of p-toluenesulfonic acid and 100 ml of methylene chloride was stirred for 30 minutes at room temperature. The reaction

mixture was neutralized with a saturated aqueous solution of sodium bicarbonate and extracted with ethyl acetate. The extract was washed with water and a saturated aqueous solution of sodium chloride, successively, dried over anhydrous sodium sulfate and concentrated under reduced pressure. The residue was purified by column chromatography on silica gel (eluent, ethyl acetate:n-hexane=6:1) to give 8.9 g of the title compound having the following physical data:

TLC(ethyl acetate:n-hexane=1:5): Rf= 0.32;

NMR: $\delta$= 7.23 (5H, bs), 4.66 (2H, s), 4.66 - 4.43 (1H, m), 3.9 - 3.1 (13H, m), 1.8 - 0.7 (41H, m) ;

IR: $\nu$= 2930, 2850, 1450, 1120 $cm^{-1}$;

MS: m/e= 562 ($M^+$), 478, 461.

By the same procedure as described in Reference Example 3, the following compound was prepared.

(a)

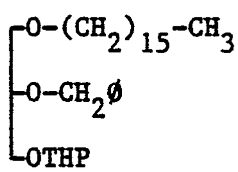

Starting material: (2RS)-1-O-hexadecyl-2-O-benzylglycerol;

TLC(ethyl acetate:n-hexane=1:5): Rf= 0.47.

Reference Example 4

Synthesis of 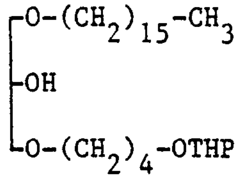

Under an atmosphere of hydrogen, a mixture of 8.6 of the benzyloxy compound (prepared in Reference Example 3), 10 g of palladium on carbon (content: 10%) and 100 ml of ethanol was stirred for two days at 50°C. The catalyst was filtered off and the filtrate was concentrated under reduced pressure to give 7.8 g of the title compound (containing impurity in part) having the following physical data:

TLC(chloroform:acetone=10:1): Rf= 0.38;

NMR: $\delta$= 4.70 - 4.45 (1H, *m*), 4.2 - 3.1 (13H, *m*), 2.0 - 0.7 (41H, *m*).

By the same procedure as described in Reference Example 4, the following compounds were prepared.

(a) 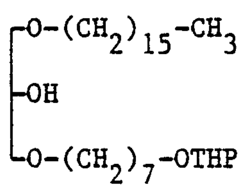

Starting material: the benzyloxy comound prepared in Reference Example 2(a);

TLC(ethyl acetate:n-hexane=1:5): Rf= 0.29.

(b) 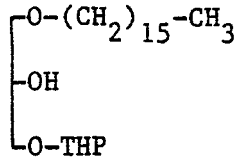

Starting material: the benzyloxy compound prepared in Reference Example 3(a);

TLC(ethyl acetate:n-hexane=1:5): Rf= 0.30.

Reference Example 5

Synthesis of 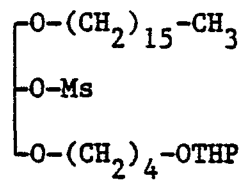

To a mixture of 7.8 g of the hydroxy compound (prepared in Reference Example 4), 8 ml of triethylamine and 150 ml of methylene chloride was added 1.4 ml of mesyl chloride under cooling with ice, and the mixture was stirred for 15 minutes. To the reaction mixture thus obtained was added 600 ml of ethyl acetate and the resulting solution was washed with water and a saturated aqueous solution of sodium chloride, successively, dried over anhydrous sodium sulfate and concentrated under reduced pressure to give 8.7 g of the crude title compound having the following physical data:

TLC(chloroform:acetone=10:1): Rf= 0.71;

NMR: $\delta$= 4.93 - 4.4 (2H, m), 3.95 - 3.1 (12H, m), 3.05 (3H, s), 2.0 - 0.7 (31H, m).

By the same procedure as described in Reference Example 5, the following compound was prepared.

(a)

$\ulcorner O-(CH_2)_{15}-CH_3$

$\vdash O-Ms$

$\llcorner O-(CH_2)_7-OTHP$

Starting material: the hydroxy compound prepared in Reference Example 4(a);

TLC(chloroform:acetone=10:1): Rf= 0.82.

Reference Example 6

Synthesis of

$\ulcorner O-(CH_2)_{15}-CH_3$

$\vdash N_3$

$\llcorner O-(CH_2)_4-OTHP$

A mixture of 8.7 g of the mesyloxy compound (prepared in Reference Example 5), 3 g of sodium azide and 100 ml of hexamethylphosphoramide (HMPA) was stirred overnight at 50°C and then to the reaction mixture was added 400 ml of ethyl acetate. The solution thus obtained was washed with water, a saturated aqueous solution of sodium bicarbonate, water and a saturated

aqueous solution of sodium chloride, successively, dried over anhydrous sodium sulfate and concentrated under reduced pressure. The residue was purified by column chromatography on silica gel (eluent, ethyl acetate:n-hexane=1:8) to give 5.5 g of the title compound having the following physical data:

TLC(ethyl acetate:n-hexane=1:5): Rf= 0.49;

NMR: $\delta$= 4.66-4.36 (1H, m), 4.0-3.1 (13H, m), 2.0-0.7 (41H, m);

IR : $\nu$= 2940, 2850, 2150, 2090, 1460, 1120 $cm^{-1}$;

MS: m/e= 469, 412, 396.

By the same procedure as described in Reference Example 6, the following compound was prepared.

(a)

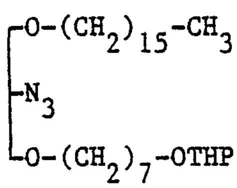

Starting material: the mesyloxy compound prepared in Reference Example 5(a);

TLC(ethyl acetate:n-hexane=1:5): Rf= 0.65;

IR : $\nu$= 2100 $cm^{-1}$.

Reference Example 7

Synthesis of

$$\begin{cases} -O-(CH_2)_{15}-CH_3 \\ -NH_2 \\ -O-(CH_2)_4-OTHP \end{cases}$$

Under an atmosphere of hydrogen, a mixture of 5.5 g of the azido compound (prepared in Reference Example 6), 2.5 g of palladium on carbon (content: 10%) and 100 ml of ethanol was stirred for 30 minutes at room temperature. The catalyst was filtered off and the filtrate was concentrated under reduced pressure to give 5.4 g of the crude title compound having the following physical data:

TLC(chloroform:methanol=10:1): Rf= 0.53;

NMR: $\delta$= 4.63 - 4.4 (1H, m), 4.0 - 3.0 (13H, m), 2.0 - 0.7 (41H, m);

IR : $\nu$= 2930, 2850, 1460, 1120 $cm^{-1}$.

By the same procedure as described in Reference Example 7, the following compounds were prepared.

(a)

$$\begin{cases} -O-(CH_2)_{15}-CH_3 \\ -NH_2 \\ -O-(CH_2)_7-OTHP \end{cases}$$

Starting material: the azido compound prepared in Reference Example 6(a);

TLC(ethyl acetate:acetic acid:water=3:1:1): Rf= 0.60.

Reference Example 8

Synthesis of 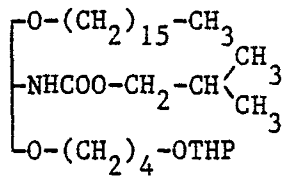

A mixture of 5.4 g of the amino compound (prepared in Reference Example 7), 2 ml of isobutyl chloroformate, 5 ml of triethylamine and 110 ml of methylene chloride was stirred for 5 minutes under cooling with ice, and then for 15 minutes at room temperature. The reaction mixture was washed with water, dried over anhydrous sodium sulfate and concentrated under reduced pressure. The residue was purified by column chromatography on silica gel (eluent, ethyl acetate:n-hexane= 1:7) to give 4.7 g of the title compound having the following physical data:

TLC (ethyl acetate:n-hexane= 1:5): Rf= 0.31;

NMR: $\delta$= 5.15-4.8 (1H, m), 4.7-4.43 (1H, m), 4.1-3.1 (15H, m), 2.0-0.7 (42H, m and 6H, d);

IR: $\nu$= 2920, 2850, 1720, 1500, 1460, 1110 $cm^{-1}$.

By the same procedure as described in Reference Example 8, the following compound was prepared.

(a)

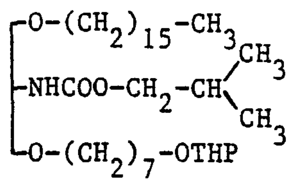

Starting material: the amino compound prepared in Reference Example 7(a);

TLC(ethyl acetate:n-hexane=1:5): Rf= 0.36.

Reference Example 9

Synthesis of

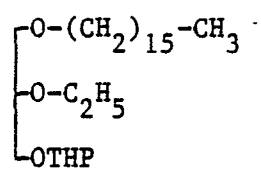

A mixture of 262 mg of the hydroxy compound [prepared in Reference Example 4(b)], 49 mg of sodium hydride (content: 63.5%) and 0.5 ml of dimethylformamide was stirred for 30 minutes at from 60°C to 70°C. After cooling to room temperature, a solution of 0.4 ml of ethyl iodide in one ml of dimethylformamide was added to the reaction mixture and the mixture was stirred for

10 minutes at the same temperature and then for one hour at 60°C. Water was added to the reaction mixture, and the solution thus obtained was extracted with ethyl acetate. The extract was washed with water and a saturated aqueous solution of sodium chloride, successively, dried over anhydrous sodium sulfate and concentrated under reduced pressure to give 296 mg of the crude title compound having the following physical data:

TLC(ethyl acetate:n-hexane=1:5): Rf= 0.48;

NMR: $\delta$= 4.7 - 4.5 (1H, m), 4.0 - 3.2 (11H, m),
2.0 - 0.6 (40H, m).

By the same procedure as described in Reference Example 9, the following compounds were prepared.

(a)

$-O-(CH_2)_{15}-CH_3$
$-O-C_2H_5$
$-O-(CH_2)_7-OTHP$

Starting material: the hydroxy compound prepared in Reference Example 4(a) and ethyl iodide;

TLC(ethyl acetate:n-hexane=1:5): Rf= 0.52;

NMR: $\delta$= 4.6 - 4.4 (1H, m), 4.0 - 3.1 (15H, m)
2.0 - 0.6 (50H, m);

MS: m/e = 542 ($M^+$), 513, 496, 459, 457
411.

(b)

$-O-(CH_2)_{15}-CH_3$
$-O-(CH_2)_2-CH_3$
$-O-(CH_2)_7-OTHP$

Starting materials: the hydroxycompound prepared in Reference Example 4(a) and propyl iodide;

TLC(ethyl acetate:n-hexane=1:5): Rf= 0.55;

NMR: $\delta$= 4.6-4.4 (1H, m), 3.9-3.1 (15H, m)
2.0-0.6 (52H, m);

MS: m/e= 556 ($M^+$), 496, 473, 471, 411.

Reference Example 10

Synthesis of

$-O-(CH_2)_{15}-CH_3$
$-O-C_2H_5$
$-OH$

A mixture of 296 mg of the tetrahydropyran-2-yloxy compound (prepared in Reference Example 9), one ml of methylene chloride, 5 ml of methanol and 10 mg of p-toluenesulfonic acid was stirred for 3 hours at room temperature. A saturated aqueous solution of sodium bicarbonate was added to the reaction mixture and the mixture thus obtained was extracted with ethyl acetate. The extract was washed with water and a saturated aqueous solution of sodium chloride, successively, dried over anhydrous sodium sulfate and concentrated under reduced pressure. The

residue was purified by column chromatography on silica gel (eluent, ethyl acetate:n-hexane=1:5) to give 165 mg of the title compound having the following physical data:

TLC(ethyl acetate:n-hexane=1:5): Rf= 0.18;

NMR: $\delta$= 3.9-3.1 (9H, m), 2.33 (1H, bs),
1.7-0.6 (34H, m);

IR : $\nu$= 3450, 2920, 2850, 1460,
1110 $cm^{-1}$.

Reference Example 11

Synthesis of 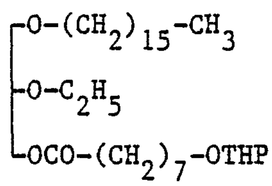

A mixture of 300 mg of the hydroxy compound (prepared in Reference Example 10), 276.6 mg of 8-(tetrahydropyran-2-yloxy)octanoic acid, 233.5 mg of dicyclohexylcarbodiimide, 4 mg of 4-dimethylaminopyridine and 10 ml of methylene chloride was stirred overnight at room temperature. Crystals precipitated from the reaction mixture were filtered off and the filtrate was concentrated under reduced pressure to give 510 mg of the crude title compound having the following physical data:

TLC(ethyl acetate:n-hexane=1:5) Rf= 0.50.

Reference Example 12

Synthesis of

$$\left[\begin{array}{l} -O-(CH_2)_{15}-CH_3 \\ -NHCOO-CH_2-CH\begin{array}{l} CH_3 \\ CH_3 \end{array} \\ -O-(CH_2)_4-OH \end{array}\right.$$

A mixture of 4.7 g of the tetrahydropyran-2-yloxy compound (prepared in Reference Example 8), 30 mg of p-toluenesulfonic acid and 100 ml of methanol was stirred for 4 hours at room temperature. To the reaction mixture was added about 30 ml of a saturated aqueous solution of sodium bicarbonate and the solution thus obtained was concentrated under reduced pressure. To the residue was added 300 ml of ethyl acetate and the solution thus obtained was washed with water and a saturated aqueous solution of sodium chloride, successively, dried over anhydrous sodium sulfate and concentrated under reduced pressure to give 4.0 g of the title compound having the following physical data:

TLC(chloroform:methanol=10:1): Rf= 0.66;

NMR: $\delta$= 5.15-4.8 (1H, m), 4.0-3.1 (11H, m), 2.2-0.7 (38H, m), 0.9 (6H, d).

By the same procedure as described in Reference Example 12, the following compounds were prepared.

Table 1

| Reference Example No. | Starting Material | Structure of Product | Physical Data of Product TLC (Rf value) (Developing Solvent) |
|---|---|---|---|
| 1 2(*a*) | Reference Example 8(a) | 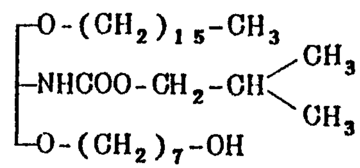 | 0.4 5 (chloroform:acetone = 10:1) |
| 1 2(*b*) | Reference Example 9(a) | 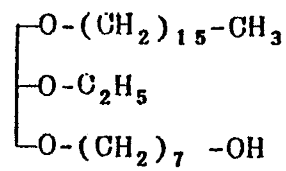 | 0.1 6 (ethyl acetate:n-hexane = 1:5) |
| 1 2(*c*) | Reference Example 9(b) | 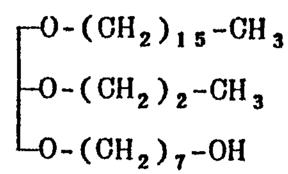 | 0.1 9 (ethyl acetate: n-hexane= 1:5) |
| 1 2(*d*) | Reference Example 2(a) | 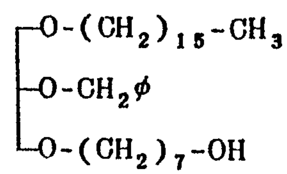 | 0.1 5 (ethyl acetate: n-hexane= 1:5) |

Reference Example 13

Synthesis of 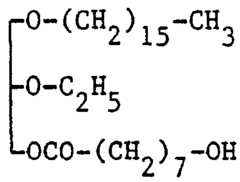

To 510 mg of the tetrahydropyran-2-yloxy compound (prepared in Reference Example 11) was added 10 ml of a mixture of acetic acid, tetrahydrofuran and water (3:1:1), and the mixture thus obtained was stirred for 5 hours at 60°C. The reaction mixture was extracted with ethyl acetate and the extract was washed with water and a saturated aqueous solution of sodium chloride, successively, dried over anhydrous sodium sulfate and concentrated under reduced pressure. The residue was purified by column chromatography on silica gel (eluent, ethyl acetate:n-hexane=1:2) to give 414 mg of the title compound having the following physical data:

TLC(chloroform:acetone=10:1) Rf= 0.54;

NMR: $\delta$= 4.3 - 4.0 (2H, m), 4.0 - 3.2 (9H, m), 2.6 - 2.0 (3H, m), 2.0 - 0.6 (44H, m);

IR: $\nu$ = 3450, 2920, 2860, 1740 $cm^{-1}$;

MS: m/e=486 ($M^+$), 468, 456, 441.

Reference Example 14

Synthesis of 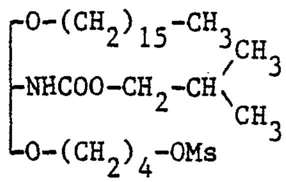

A mixture of 1.5 g of the hydroxy compound (prepard in Reference Example 12), 0.28 ml of mesyl chloride, 1.5 ml of triethylamine and 15 ml of methylene chloride was stirred for one hour under cooling with ice. The reaction mixture was washed with water, a saturated aqueous solution of sodium bicarbonate and water, successively, dried over anhydrous sodium sulfate and concentrated under reduced pressure. The residue was purified by column chromatography on silica gel (eluent, ethyl acetate: n-hexane=1:3) to give 1.65 g of the title compound having the following physical data:

TLC(ethyl acetate:n-hexane=1:2): Rf=0.33;

NMR: $\delta$= 5.15 - 4.8 (1H, m), 4.2 (2H, t),
4.0 - 3.2 (11H, m), 3.0 (3H, s),
2.1 - 0.7 (36H, m), 0.93 (6H, d);

IR : $\nu$=2930, 2860, 1720, 1510, 1470,
1360, 1180, 1120 $cm^{-1}$.

By the same procedure as described in Reference Example 14, the following compounds were prepared.

Table 2

| Reference Example No. | Starting Material | Structure of Product | Physical Data of Product | | |
|---|---|---|---|---|---|
| | | | TLC (Rf value) (Developing Solvent) | NMR ($\delta$) | MS($m/e$) |
| 14(a) | Reference Example 12(a) | $-O-(CH_2)_{15}-CH_3$<br>$-NHCOO-CH_2-CH(CH_3)_2$<br>$-O-(CH_2)_7-OMs$ | 0.70 (chloroform: acetone= 10:1) | 5.1 - 4.8 (1H, m), 4.18 (2H, t), 4.0 - 3.1 (11H, m), 3.96 (3H, s), 2.0 - 0.7 (42H, m), 0.93 (6H, d) | |
| 14(b) | Reference Example 12(b) | $-O-(CH_2)_{15}-CH_3$<br>$-O-C_2H_5$<br>$-O-(CH_2)_7-OMs$ | 0.84 (chloroform: acetone= 10:1) | 4.16 (2H, t), 3.58 (2H, q) 3.6 - 3.2 (9H, m), 2.96 (3H, s), 2.0 - 0.7 (44H, m) | 537, 536(M$^+$), 507, 490 |
| 14(c) | Reference Example 12(c) | $-O-(CH_2)_{15}-CH_3$<br>$-O-(CH_2)_2-CH_3$<br>$-O-(CH_2)_7-OMs$ | 0.88 (chloroform: acetone= 10:1) | 4.16 (2H, t), 3.8 - 3.2 (11H, m), 2.96 (3H, s), 2.0 - 0.7 (46H, m) | 551, 550(M$^+$), 507, 490 |
| 14(d) | Reference Example 12(d) | $-O-(CH_2)_{15}-CH_3$<br>$-O-CH_2\phi$<br>$-O-(CH_2)_7-OMs$ | 0.81 (chloroform: acetone= 10:1) | 7.2 (5H, s), 4.63 (2H, s), 4.13 (2H, t), 3.8 - 3.2 (9H, m), 2.9 (3H, s), 2.0 - 0.7 (41H, m) | 598(M$^+$) |
| 14(e) | Reference Example 13 | $-O-(CH_2)_{15}-CH_3$<br>$-O-C_2H_5$<br>$-OCO-(CH_2)_7-OMs$ | 0.76 (chloroform: acetone: 10:1) | | |

Table 2 (continued)

| Reference Example No. | Starting Material | Structure of Product | Physical Data of Product | | |
|---|---|---|---|---|---|
| | | | TLC (Rf value) (Developing Solvent) | NMR (δ) | MS (m/e) |
| 14(f) | Reference Example 2 | $-O-(CH_2)_{15}-CH_3$<br>$-O-CH_2\phi$<br>$-O-(CH_2)_4-OMs$ | 0.67 (chloroform: acetone= 10:1) | 7.2 (5H, bs), 4.6 (2H, s), 4.2 (2H, bt), 3.8 - 3.1 (9H, m), 2.9 (3H, s), 2.0 - 0.8 (35H, m) | |

Example 1

Synthesis of 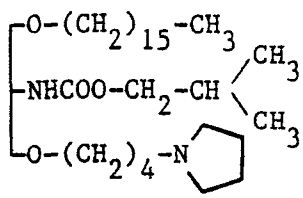

A mixture of 114 mg of the mesyloxy compound (prepared in Reference Example 14), 0.1 ml of pyrrolidine and 3 ml of dimethylformamide was stirred for 1.5 hours at 70°C and then the reaction mixture was concentrated under reduced pressure. The residue was purified by column chromatography on silica gel (eluent, chloroform:methanol:triethylamine=30:1:0.01) to give 106 mg of the title compound having the following physical data:

TLC(ethyl acetate:acetic acid:water=3:1:1) Rf= 0.83;

NMR: $\delta$= 5.11-4.95(1H, m), 3.96-3.78(3H, m), 3.59-3.35(8H, m), 2.70-2.46 (6H, m), 1.95-1.76(5H, m), 1.70 -1.46(6H, m), 1.25(26H, m), 0.92(6H, d), 0.87(3H, m);

IR : $\nu$= 2930, 2860, 1730, 1500, 1460, 1200, 1120 $cm^{-1}$;

MS: m/e= 540($M^+$), 467, 424, 315.

By the same procedure as described in Example 1, the following compounds were prepared.

Table 3 (1)

| Example No. | Starting Material | Structure of Product | Physical Data of Product | | | |
|---|---|---|---|---|---|---|
| | | | TLC (Rf value) (Developing Solvent) | NMR (δ) | IR (ν) | MS(m/e) |
| 1 (a) | Compound prepared in Reference Example 14 and piperidine | 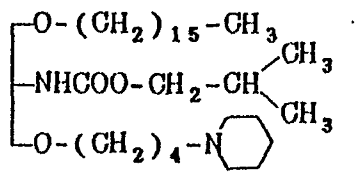 | 0.85 (ethyl acetate: acetic acid: water: 3:1:1) | 5.10 - 4.95 (1H, m), 3.94 - 3.78 (3H, m), 3.58 - 3.36 (8H, m), 2.48 - 2.25 (6H, m), 2.00 - 1.40 (13H, m), 1.25 (26H, m), 0.92 (6H, d), 0.87 (3H, m) | 2930, 2860, 1730, 1500, 1470, 1200, 1120 | 554($M^+$) 481, 438, 329 |
| 1(b) | Compound prepared in Reference Example 14 and quinuclidine | 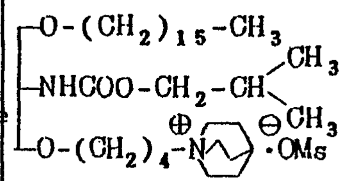 | 0.67 (ethyl acetate: acetic acid: water: 3:1:1) Melting Poing: 36-39°C | 5.38 - 5.26 (1H, m), 3.96 - 3.72 (3H, m), 3.70 - 3.35 (16H, m), 2.77 (3H, s), 2.28 - 2.17 (1H, m), 2.15 - 1.98 (6H, m), 1.95 - 1.45 (7H, m), 1.25 (26H, m), 0.92 (6H, d), 0.88 (3H, m) | 3500, 2940, 2860, 1710, 1470, 1200, 1120 | |
| 1 (c) | Compound prepared in Reference Example 14 and thiazole | 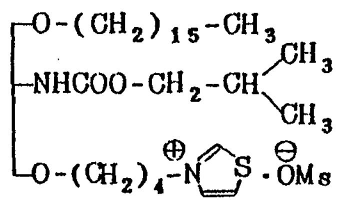 | 0.56 (ethyl acetate: acetic acid: water= 3:1:1) | 11.22 (1H, m), 8.44 (1H, m), 8.09 (1H, m), 5.40 - 5.22 (1H, m), 4.00 - 3.74 (3H, m), 3.60 - 3.36 (8H, m), 2.80 (3H, s), 2.26 - 2.05 (2H, m), 1.98 - 1.46 (5H, m), 1.26 (26H, m), 0.90 (6H, d), 0.87 (3H, m) | 3400, 2930, 2850, 1700, 1120 | 554, 436, 397, 384 |
| 1 (d) | Compound prepared in Reference Example 14 and pyridine | 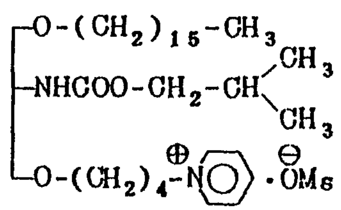 | 0.53 (ethyl acetate: acetic acid: water= 3:1:1) | 9.52 - 9.38 (2H, m), 2.50 - 2.36 (1H, m), 8.17 - 8.02 (2H, m), 5.36 - 5.25 (1H, m), 5.07 - 4.92 (2H, m), 4.00 - 3.35 (11H, m), 2.79 (3H, s), 2.30 - 2.12 (2H, m), 2.00 - 1.45 (5H, m), 1.26 (26H, m), 0.90 (6H, d), 2.00- 0.82 (3H, m) | 3400, 2920, 2850, 1700, 1110 | 549($M^+$) 384 |

Table 3 (2)

| Example No. | Starting Material | Structure of Product | Physical Data of Product: TLC (Rf value (Developing Solvent)) | NMR (δ) | IR (ν) | MS(m/e) |
|---|---|---|---|---|---|---|
| 1 (e) | Compound prepared in Reference Example 14 and 1-methyl-imidazole | 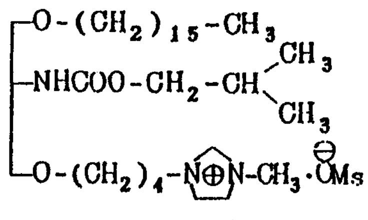 | 0.68 (ethyl acetate: acetic acid:water= 3:1:1) Melting Point: 30-31°C | 10.03(1H, bs)、7.29(1H, bs) 7.10(1H, bs)、5.34-5.22(1H m)、4.34(2H, t)、4.05(3H, s) 3.98-3.75(3H, m)、3.59-3.35 (8H, m)、2.79(3H, s)、2.10-1.46(7H, m)、1.26(26H, m)、0.91(6H, d)、0.87(3H, m) | 3450, 2920、2850、1690、1540、1470 1190、1120、 | 537、464、421 |
| 1 (f) | Compound prepared in Reference Example 14(a) and thiazole | 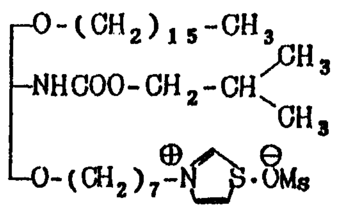 | 0.66 (ethyl acetate: acetic acid: water= 3:1:1) | 11.15-11.05(1H, m)、8.30-8.14(2H, m)、5.1-5.0(1H, m) 4.75(2H, t)、3.95-3.76(3H, m)、3.4-3.3(8H, m)、2.79(3H s)、2.1-1.2(39H, m)、0.91(6H, d)、0.87(3H, m) | 3400、3320、2920、2850、1690、1120 | 596、511、478、397 |
| 1 (g) | Compound prepared in Reference Example 14(a) and pyridine | 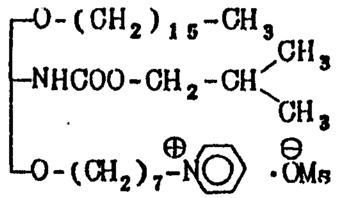 | 0.60 (ethyl acetate: acetic acid: water = 3:1:1) | 9.25-9.13(2H, m)、8.50-8.37 (1H, m)、8.16-8.0(2H, m)、5.10-4.98(1H, m)、4.84(2H, t)、3.90-3.76(3H, m)、3.56-3.3(8H, m)、2.76(3H, s)、2.1-1.15(39H, m)、0.91(6H, d) 0.87(3H, m) | 3450、3320、2930、2850、1695、1120 | 591($M^+$)、516、397、 |
| 1 (h) | Compound prepared in Reference Example 14(b) and thiazole | 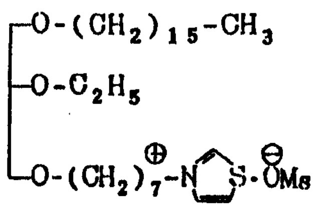 | 0.48 (ethyl acetate: acetic acid: water = 3:1:1) | 11.05(1H, bs)、8.21(2H, m)、4.74(2H, m)、3.63(2H, q)、3.6-3.35(9H, m)、2.79(3H, s)、2.1-1.2(38H, m)、1.19 (3H, t)、0.88(3H, m) | 3450、2920、2850、1470、1110 | |

Table 3 (3)

| Example No. | Starting Material | Structure of Product | Physical Data of Product: TLC (Rf value) (Developing Solvent) | NMR (δ) | IR (ν) | MS(m/e) |
|---|---|---|---|---|---|---|
| 1 (*i*) | Compound prepared in Reference Example 14(c) and thiazole | 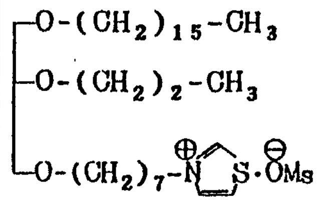 | 0.72 (ethyl acetate: acetic acid: water = 3:1:1) | 11.19-11.08(1H, *m*), 8.28-8.14(2H, *m*), 4.74(2H, *t*), 3.64-3.32(11H, *m*), 2.79(3H, *s*), 2.12-1.18(40H, *m*), 0.90(3H, *t*), 0.87(3H, *m*) | 3450, 2920, 2850, 1470, 1120 | 540($M^+$), 512, 480 |
| 1 (*j*) | Compound prepared in Reference Example 14(d) and pyrrolidine | 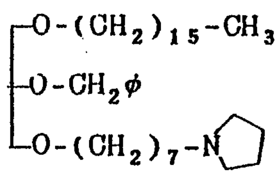 | 0.58 (ethyl acetate: acetic acid: water = 3:1:1) | 7.13(5H, *s*), 4.53(2H, *s*), 3.8-3.0(9H, *m*), 2.7-2.2(6H, *m*), 2.0-0.7(45H, *m*) | | 573($M^+$), 482, 466 |
| 1 (*k*) | Compound prepared in Reference Example 14(d) and thiazole | 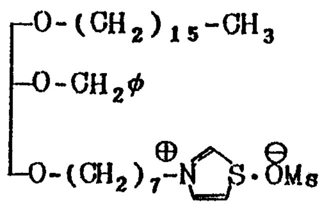 | 0.52 (ethyl acetate: acetic acid: water = 3:1:1) | 11.3-11.2(1H, *m*), 8.20-8.08(2H, *m*), 7.40-7.26(5H, *m*), 4.67(2H, *s*), 4.80-4.65(2H, *m*), 3.79-3.62(1H, *m*) 3.60-3.34(8H, *m*), 2.79(3H, *bs*), 2.10-1.18(38H, *m*), 0.87(3H, *m*) | 3450, 3060, 2930, 2860, 1470, 1120 | 588($M^+$) |
| 1 (*ℓ*) | Compound prepared in Reference Example 14(e) and thiazole | 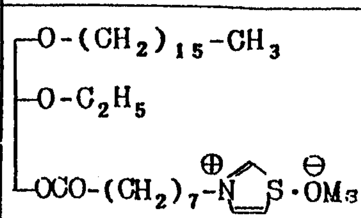 | 0.69 (ethyl acetate: acetic acid: water = 3:1:1) | 10.96(1H, *m*), 8.2(2H, *m*), 4.72(2H, *t*), 4.3-4.0(2H, *m*), 3.70-3.55(1H, *m*), 3.62(2H, *q*), 3.52-3.38(4H, *m*), 2.79(3H, *s*), 2.32(2H, *t*), 2.0-1.2(38H, *m*), 1.19(3H, *t*), 0.88(3H, *m*) | 3450, 2920, 2850, 1740, 1470, 1110 | |

Table 3 (4)

| Example No. | Starting Material | Structure of Product | Physical Data of Product: TLC (Rf value) (Developing Solvent) | NMR (δ) | IR (ν) | MS(m/e) |
|---|---|---|---|---|---|---|
| 1 (m) | Compound prepared in Reference Example 14(f) and quinuclidine | 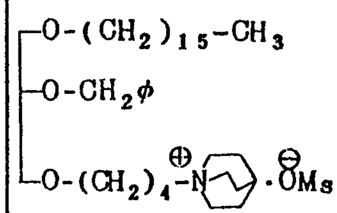 | 0.58 (ethyl acetate: acetic acid: water = 3:1:1) | 7.42-7.25(5H,m), 4.65(2H,q), 3.80-3.66(1H,m), 3.63-3.30(16H,m), 2.77(3H,s), 2.20-2.10(1H,m), 2.02-1.48(12H,m), 1.26(26H,m), 0.88(3H,m) | 3450, 2930, 2850, 1460, 1200, 1110 | |
| 1 (n) | Compound prepared in Reference Example 14(f) and triethylene-diamine | 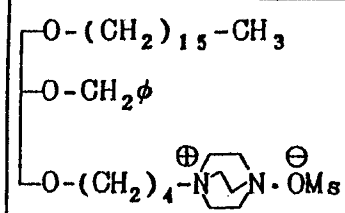 | 0.48 (ethyl acetate: acetic acid: water = 3:1:1) | 7.43-7.27(5H,m), 4.67(2H,q), 3.82-3.69(1H,m), 3.65-3.0(22H,m), 2.76(3H,s), 1.93-1.49(6H,m), 1.26(26H,m), 0.88(3H,m) | 3400, 2930, 2860, 1460, 1210 | |
| 1 (o) | Compound prepared in Reference Example 14(f) and 1-methyl-imidazole | 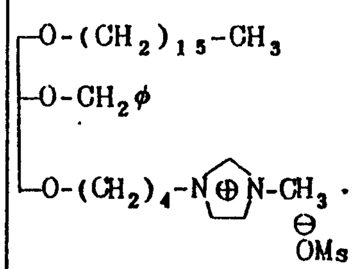 | 0.58 (ethyl acetate: acetic acid: water = 3:1:1) | 10.05(1H,bs), 7.38-7.26(5H,m), 7.18(1H,bs), 7.00(1H,bs), 4.65(2H,q), 4.3(2H,t), 3.97(3H,s), 3.98-3.66(1H,m), 3.61-3.36(8H,m), 2.79(3H,s), 2.10-1.91(2H,m), 1.70-1.46(4H,m), 1.25(26H,m), 0.87(3H,m) | 3450, 2940, 2860, 1570, 1470, 1200, 1120 | 528, 437, 421 |

Table 3 (5)

| Example No. | Starting Material | Structure of Product | Physical Data of Product: TLC (Rf value) (Developing Solvent) | Physical Data of Product: NMR (δ) | Physical Data of Product: IR (ν) | Physical Data of Product: MS(m/e) |
|---|---|---|---|---|---|---|
| 1 (p) | Compound prepared in Reference Example 2(b) and pyrrolidine | 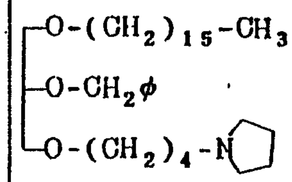 | 0.79 (ethyl acetate: acetic acid: water = 3:1:1) | 7.43-7.24(5H,m), 4.67(2H,s), 3.78-3.62(1H,m), 3.60-3.35(8H,m), 3.80-3.59(6H,m), 1.94-1.82(4H,m), 1.78-1.46(6H,m), 1.25(26H,m), 0.87(3H,m) | 3400, 2930, 2860, 1450, 1120 | 531($M^+$) 440, 424 |
| 1 (q) | Compound prepared in Reference Example 2(b) and piperidine | 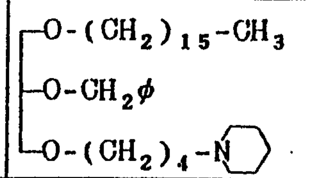 | 0.71 (ethyl acetate: acetic acid: water = 3:1:1) | 7.40-7.24(5H,m), 4.68(2H,s), 3.73-3.64(1H,m), 3.60-3.36(8H,m), 2.45-2.22(6H,m), 1.86-1.37(12H,m), 1.25(26H,m), 0.88(3H,m) | 2930, 2850, 1460, 1110 | 545($M^+$) 454, 438 |
| 1 (r) | Compound prepared in Reference Example 2(b) and hexamethyleneimine | 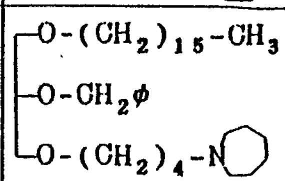 | 0.78 (ethyl acetate: acetic acid: water = 3:1:1) | 7.44-7.24(5H,m), 4.68(2H,s), 3.80-3.63(1H,m), 3.62-3.36(8H,m), 2.85-2.55(6H,m), 1.84-1.45(14H,m), 1.26(26H,m), 0.88(3H,m) | 2930, 2860, 1450, 1120 | 559($M^+$) 468, 452 |
| 1 (s) | Compound prepared in Reference Example 2(b) and piperazine | 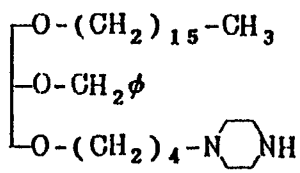 | 0.64 (ethyl acetate: acetic acid: water = 3:1:1) | | | 546($M^+$) 518, 504, 455, 439 |
| 1 (t) | Compound prepared in Reference Example 2(b) and morpholine | 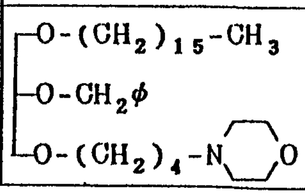 | 0.79 (ethyl acetate: acetic acid: water = 3:1:1) | | | 547($M^+$) 456, 440 |

Example 2

Synthesis of 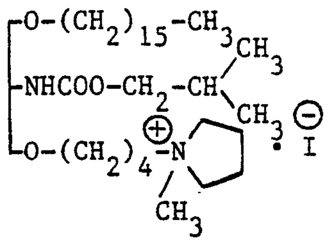

A mixture of 93 mg of the pyrrolidinyl compound (prepared in Example 1), 0.3 ml of methyl iodide and 3 ml of methanol was stirred overnight at room temperature and then the reaction mixture was concentrated under reduced pressure. The residue was purified by column chromatography on silica gel (eluent, chloroform:methanol=10:1 → chloroform:methanol:water=65:35:5) to give 106 mg of the title compound having the following physical data:

TLC(ethyl acetate:acetic acid:water=3:1:1) Rf= 0.58;

NMR: δ= 5.20-5.06 (1H, m), 3.98-3.37 (17H, m), 3.27 (3H, s), 2.42-2.25 (4H, m), 2.03-1.45 (7H, m), 1.26 (26H, m), 0.93 (6H, d), 0.88 (3H, m);

IR : ν= 3470, 2920, 2850, 1700, 1460, 1110 $cm^{-1}$;

MS: m/e= 540, 467.

By the same procedure as described in Example 2, the following compounds were prepared.

Table 4 (1)

| Example No. | Starting Material | Structure of Product | Physical Data of Product | | | |
|---|---|---|---|---|---|---|
| | | | TLC (Rf value) (Developing Solvent) | NMR (δ) | IR (ν) | MS (m/e) |
| 2 (*a*) | Example 1(a) | 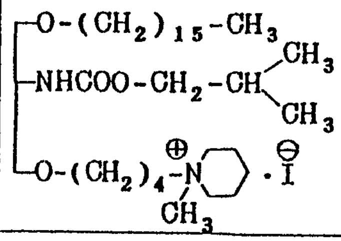 | 0.65 (ethyl acetate: acetic acid: water = 3:1:1) | 5.15−5.02 (1H, *m*), 3.96−3.36 (17H, *m*), 3.30 (3H, *s*), 2.05 −1.46 (13H, *m*), 1.26 (26H, *m*), 0.92 (6H, *d*), 0.88 (3H, *m*) | 3500, 2930, 2860, 1700, 1470, 1120 | 554, 481, 384 |
| 2 (*b*) | Example 1(j) | 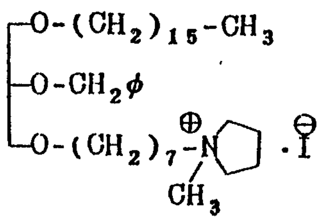 | 0.55 (ethyl acetate: acetic acid: water = 3:1:1) | 7.2 (5H, *s*), 4.6 (2H, *s*), 3.9−3.1 (13H, *m*), 3.03 (3H, *s*), 2.4−2.0 (4H, *m*), 1.9−0.7 (45H, *m*) | | 573, 482, 466 |
| 2 (*c*) | Example 1(p) | 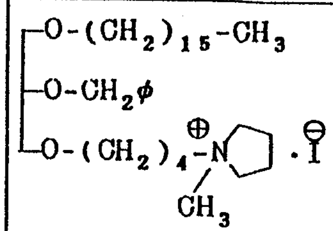 | 0.58 (ethyl acetate: acetic acid: water = 3:1:1) Melting Point: 40-43°C | 7.40−7.25 (5H, *m*), 7.66 (2H *q*), 3.80−3.37 (15H, *m*), 3.09 (3H, *s*), 2.29−2.09 (4H *m*), 2.00−1.47 (6H, *m*), 1.25 (26H, *m*), 0.87 (3H, *m*) | 3450, 2930, 2860, 1470, 1120 (KBr method) | 531, 440, 424 |
| 2 (*d*) | Example 1(q) | 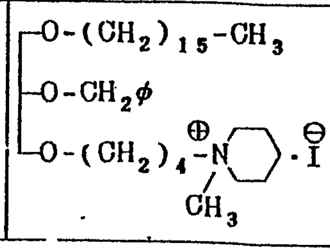 | 0.60 (ethyl acetate: acetic acid: water = 3:1:1) Melting Point: 65-69°C | 7.42−7.25 (5H, *m*), 4.66 (2H *q*), 3.80−3.35 (15H, *m*), 3.14 (3H, *s*), 1.97−1.45 (12H, *m*), 1.25 (26H, *m*), 0.87 (3H, *m*) | 2920, 2850, 1470, 1120 (KBr method) | 588, 545, 454, 438 |

Table 4 (2)

| Example No. | Starting Material | Structure of Product | Physical Data of Product | | | |
|---|---|---|---|---|---|---|
| | | | TLC (Rf value) (Developing Solvent) | NMR (δ) | IR (ν) | MS (m/e) |
| 2 (e) | Example 1(r) | 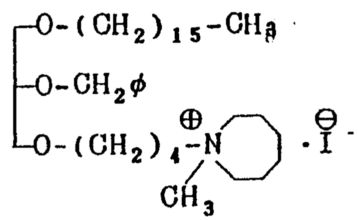 | 0.64 (ethyl acetate: acetic acid: water = 3:1:1) | 7.42-7.25(5H, m), 4.66(2H q), 3.80-3.37(15H, m), 3.16(3H, s), 2.00-1.47 (14H, m), 1.25(26H, m), 0.87(3H, m) | 2930, 2860, 1470, 1110 | 588, 574(M⁺) 559, 504, 468, 452 |
| 2 (f) | Example 1(s) | 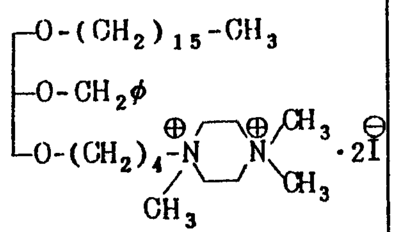 | 0.53 (ethyl acetate: acetic acid: water = 3:1:1) | 7.41-7.24(5H, m), 4.66 (2H, s), 3.80-3.14(28H, m), 1.70-1.43(6H, m), 1.24(26H, m), 0.87(3H, m) (CDCl$_3$ + CD$_3$OD solution) | 3400, 2930, 2860, 1650, 1460, 1110 | 560, 518, 504 |
| 2 (g) | Example 1(t) | 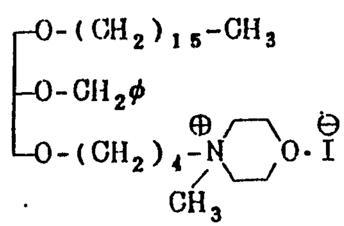 | 0.60 (ethyl acetate: acetic acid: water = 3:1:1) | 7.42-7.25(5H, m), 4.66 (2H, q), 3.97-3.66(7H, m), 3.66-3.37(12H, m), 3.33(3H, s), 1.98-1.46 (6H, m), 1.25(26H, m), 0.88(3H, m) | 2920, 2850, 1460, 1110 | 588, 547, 456, 440 |

Example 3

Synthesis of 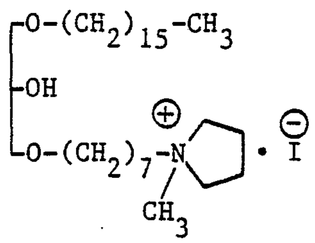

By the same procedure as described in Reference Example 4, the title compound having the following physical data was prepared from the benzyloxy compound [prepared in Example 2(b)].

NMR: δ = 4.0 - 3.3 (15H, m), 3.26 (3H, s),
2.5 - 2.3 (4H, m), 2.0 - 0.7 (45H, m).

Example 4

Synthesis of 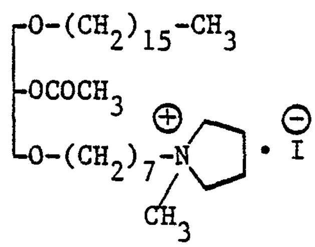

A mixture of 54 mg (containing impurity in part) of the hydroxy compound (prepared in Example 3), 0.5 ml of acetic anhydride and 7 ml of pyridine was stirred overnight at 40°C and then the reaction mixture was concentrated under reduced pressure. The residue was purified by column chromatography on silica gel (eluent, chloroform:methanol=10:1 → chloroform:methanol:water=65:35:2) to give 65 mg of the title compound having the following physical data:

TLC(ethyl acetate:acetic acid:water=3:1:1): Rf= 0.45;

NMR: δ =5.20-5.06 (1H, m), 3.91-3.75 (4H, m), 3.75-3.34 (10H, m), 3.27 (3H, s), 2.40-2.22 (4H, m), 2.08 (3H, s), 1.90-1.18 (38H, m), 0.87 (3H, m);

IR : ν= 3450, 2920, 2850, 1730, 1470, 1120 $cm^{-1}$;

MS: m/e= 525, 455.

Example 5

Synthesis of

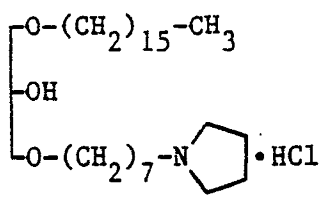

By the same procedure as described in Reference Example 4, the title compound having the following physical data was prepared from the benzyloxy compound [prepared in Example 1(j)].

TLC(ethyl acetate:acetic acid:water=3:1:1): Rf= 0.58;

IR : ν= 3400, 2930, 2850, 1110 $cm^{-1}$;

MS: m/e= 483, 455.

Reference Example 15

 

Synthesis of 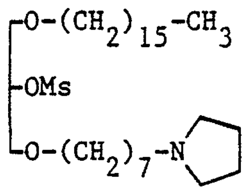

By the same procedure as described in Reference Example 5, the title compound having the following physical data was prepared from the hydroxy compound (prepared in Example 5).

TLC(ethyl acetate:acetic acid:water=3:1:1): Rf= 0.73;

NMR: $\delta$= 4.9 - 4.5 ( 1H, m )、3.66 - 3.1 ( 8H, m )
3.0 ( 3H, s )、2.66 - 2.2 ( 6H, m )、
2.0 - 0.7 ( 45H, m ).

Example 6

Synthesis of 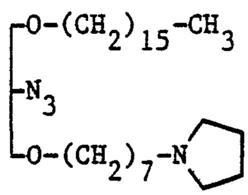

By the same procedure as described in Reference Example 6, the title compound having the following physical data was prepared from the mesyloxy compound (prepared in Reference Example 15).

TLC(ethyl acetate:acetic acid:water=3:1:1): Rf= 0.77;

NMR: $\delta$= 3.7 - 3.1 ( 9H, m )、2.7 - 2.1 ( 6H, m )、
2.0 - 0.7 ( 45H, m );

IR : $\nu$= 2930、2860、2100、1460、
1120 $cm^{-1}$;
MS: m/e= 508($M^+$)、466.

Example 7

Synthesis of 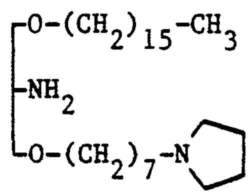

A mixture of 410 mg of the azido compound (prepared in Example 6), 148 mg of lithium aluminium hydride and 20 ml of diethyl ether was refluxed for one hour. After cooling to room temperature, a 10% aqueous solution of sodium hydroxide was added to the reaction mixture and the mixture was filtered. The filtrate was concentrated under reduced pressure to give 314 mg of the title compound having the following physical data:
TLC(ethyl acetate:acetic acid:water=3:1:1): Rf= 0.55;
MS: m/e= 482($M^+$), 412.

Example 8

Synthesis of 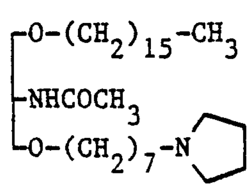

A mixture of 111 mg of the amino compound (prepared in Example 7), 0.1 ml of acetic anhydride, 0.5 ml of triethylamine and 5 ml of methylene chloride was stirred for 2 hours at room temperature and then the reaction mixture was concentrated under reduced pressure. The residue was purified by column chromatography on silica gel (eluent, chloroform:methanol: triethylamine=30:1:0.05) to give 111 mg of the title compound having the following physical data:

TLC(ethyl acetate:acetic acid:water=3:1:1) Rf= 0.75;

NMR: $\delta$= 5.9 - 5.6 (1H, *m*), 4.4 - 3.8 (1H, *m*),
3.7 - 3.1 (8H, *m*), 3.0 - 2.4 (6H, *m*),
1.93 (3H, *s*), 2.0 - 0.7 (45H, *m*);

IR : $\nu$= 3300, 2930, 2860, 1660, 1470
1120 $cm^{-1}$;

MS: *m/e*= 524($M^+$), 481, 466, 454.

Example 9

Synthesis of 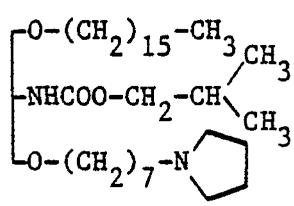

By the same procedure as described in Reference Example 8, the title compound having the following physical data was prepared from the amino compound (prepared in Example 7).

TLC(ethyl acetate:acetic acid:water=3:1:1): Rf= 0.81;

NMR: $\delta$= 5.1 - 4.7 ( 1 H, *m* )、4.0 - 3.1 ( 11 H, *m* )
2.8 - 2.2 ( 6 H, *m* )、2.0 - 0.6 ( 46 H, *m* )
0.86 ( 6 H, *d* ) ;

IR : $\nu$= 3480、2930、2850、1720、
1120 $cm^{-1}$;

MS: *m/e*= 582 ( $M^{+}$ )、509、481、466.

Example 10

Synthesis of 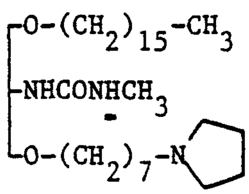

A mixture of 103 mg of the amino compound (prepared in Example 7), 30 µl of methyl isocyanate, 0.1 ml of triethylamine and 5 ml of methylene chloride was stirred for 2 hours at room temperature and then the reaction mixture was concentrated under reduced pressure. The residue was purified by column chromatography on silica gel (eluent, chloroform:methanol:triethylamine=30:1:0.05) to give 112 mg of the title compound having the following physical data:

TLC(ethyl acetate:acetic acid:water=3:1:1): Rf= 0.75;

NMR: $\delta$= 5.3 - 4.7 ( 2 H, *m* )、4.1 - 3.6 ( 1 H, *m* )、
3.6 - 3.1 ( 8 H, *m* )、2.7 ( 3 H, *d* )、2.8 -
2.3 ( 6 H, *m* )、2.0 - 0.7 ( 45 H, *m* ) ;

IR : $\nu$= 3350、2930、2860、1630、1580、

1470、1120 $cm^{-1}$;

MS: m/e= 539($M^+$)、508.

Example 11

By the same procedure as described in Example 2, the following compounds were prepared.

Table 5

| Example No. | Starting Material | Structure of Product | Physical Data of Product | | | |
|---|---|---|---|---|---|---|
| | | | TLC (Rf value) (Developing Solvent) | NMR (δ) | IR (ν) | MS (m/e) |
| 11(a) | Example 8 | 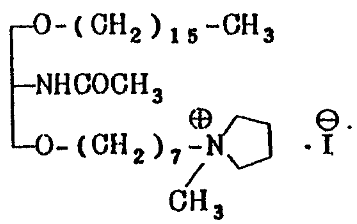 | 0.46 (ethyl acetate: acetic acid: water = 3:1:1) | 6.02-5.92(1H, m), 4.24-4.10(1H, m), 3.93-3.34(14H, m), 3.27(3H, s), 2.42-2.22(4H, m), 1.99(3H, s), 1.90-1.16(38H, m), 0.87(3H, m) | 3450, 3300, 2920, 2850, 1650, 1460, 1130 | 524, 466 |
| 11(b) | Example 9 | 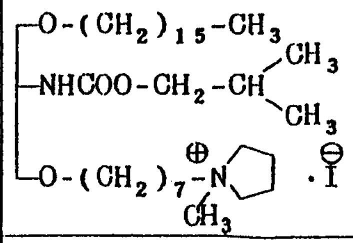 | 0.54 (ethyl acetate: acetic acid: water = 3:1:1) | 5.08-4.95(1H, m), 3.95-3.74(7H, m), 3.72-3.57(2H, m), 3.57-3.35(8H, m), 3.27(3H, s), 2.40-2.22(4H, m), 2.00-1.18(39H, m), 0.92(6H, d), 0.87(3H, m) | 3500, 3300, 2930, 2850, 1710, 1460, 1120 | 582, 509 |
| 11(c) | Example 10 | 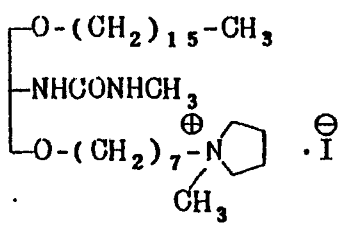 | 0.44 (ethyl acetate: acetic acid: water = 3:1:1) Melting Point: 53-58°C | 5.50-5.38(1H, m), 5.12(1H, bd), 4.00-3.60(7H, m), 3.77-3.33(8H, m), 3.25(3H, s), 2.73(3H, d), 2.44-2.20(4H, m), 1.94-1.15(38H, m), 0.87(3H, m) | 3350, 2930, 2850, 1630, 1580, 1470, 1120 | 539, 508 469, 466 |

Reference Example 16

Synthesis of 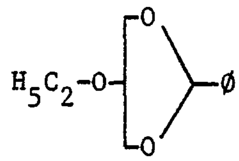

By the same procedure as described in Reference Example 9, the title compound having the following physical data was prepard from 1,3-O-benzylidene glycerol (the compound is described in J. Amer. Chem. Soc., 50, 2235 (1928)) and ethyl iodide.

TLC(chloroform:acetone=10:1): Rf= 0.71.

Reference Example 17

Synthesis of 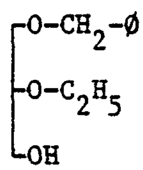

To a solution of 5.8 g of the benzylidene compound (prepared in Reference Example 16) in 15 ml of toluene was added dropwise 28 ml of diisobutylaluminium hydride under cooling with ice and the mixture was stirred for 1.5 hours at room temperature. To the reaction mixture were added methanol and water, and the mixture was filtered. The filtrate was concentrated under reduced pressure to give 7 g of the crude title compound having the following physical data:

TLC(chloroform:acetone=10:1): Rf= 0.34;

NMR: $\delta$= 7.3(5H,s), 4.55(2H,s), 4.0-3.3(7H,m), 1.1(3H,t);

MS: m/e= 210($M^+$), 179, 119.

Reference Example 18

By the same procedure as described in Reference Example 2, the following compounds were prepared.

(a)

$-O-CH_2-\phi$
$-O-C_2H_5$
$-O-(CH_2)_6-OTHP$

Starting materials: the hydroxy compound prepared in Reference Example 17 and 6-(tetrahydropyran-2-yloxy)-1-mesyloxyhexane;

TLC(chloroform:acetone=10:1): Rf= 0.67.

(b)

$-O-(CH_2)_{15}-CH_3$
$-O-C_2H_5$
$-O-(CH_2)_6-OTHP$

Starting materials: the hydroxy compound prepared in Reference Example 10 and 6-(tetrahydropyran-2-yloxy)-1-mesyloxyhexane;

TLC(ethyl acetate:n-hexane=1:5): Rf= 0.56.

0146258

(c)

$-O-(CH_2)_{15}-CH_3$

$-O-C_2H_5$

$-O-(CH_2)_6-Br$

Starting materials: the hydroxy compound prepared in Reference Example 10 and 1,6-dibromohexane;

TLC(ethyl acetate:n-hexane=1:5): Rf= 0.52.

(d)

$-O-(CH_2)_{15}-CH_3$

$-O-C_2H_5$

$-O-(CH_2)_8-Br$

Starting materials: the hydroxy compound prepared in Reference Example 10 and 1,8-dibromooctane;

TLC(ethyl acetate:n-hexane=1:5): Rf= 0.59.

Reference Example 19

Synthesis of

$-OH$

$-O-C_2H_5$

$-O-(CH_2)_6-OTHP$

By the same procedure as described in Reference Example 4, the title compound having the following physical data was prepared from the benzyloxy compound prepared in Reference Example 18(a).

0146258

TLC(chloroform:acetone=10:1): Rf= 0.23;

NMR: $\delta$= 4.7-4.5(1H,m), 4.1-3.2(13H,m);

MS: m/e= 303, 219, 201.

Reference Example 20

Synthesis of

$-O-(CH_2)_{21}-CH_3$
$-O-C_2H_5$
$-O-(CH_2)_6-OTHP$

By the same procedure as described in Reference Example 2, the title compound having the following physical data was prepared from the hydroxy compound prepared in Reference Example 19 and 1-mesyloxydocosane.

TLC(chloroform:acetone=10:1): Rf= 0.71.

Reference Example 21

By the same procedure as described in Reference Example 12, the following compounds were prepared.

(a)

$-O-(CH_2)_{21}-CH_3$
$-O-C_2H_5$
$-O-(CH_2)_6-OH$

Starting material: the tetrahydropyran-2-yloxy compound prepared in Reference Example 20;

TLC(chloroform:acetone=10:1): Rf= 0.38;

NMR: $\delta$ =3.8-3.3(13H,m), 1.8-0.7(54H,m);

MS: m/e= 528($M^+$), 482, 381.

(b)

$-O-(CH_2)_{15}-CH_3$

$-O-C_2H_5$

$-O-(CH_2)_6-OH$

Starting material: the tetrahydropyran-2-yloxy compound prepared in Reference Example 18(b);

TLC(ethyl acetate:n-hexane=1:5): Rf= 0.20.

Reference Example 22

By the same procedure as described in Reference Example 14, the following compounds were prepared.

(a)

$-O-(CH_2)_{21}-CH_3$

$-O-C_2H_5$

$-O-(CH_2)_6-OMs$

Starting material: the hydroxy compound prepared in Reference Example 21(a);

TLC(chloroform:acetone=10:1): Rf= 0.67;

NMR: $\delta$= 4.5-4.0(2H,m), 3.8-3.3(11H,m), 3.0(3H,s).

(b)

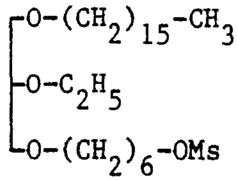

Starting material: the hydroxy compound prepared in Reference Example 21(b);

TLC(chloroform:acetone=10:1): Rf= 0.73.

Example 12

By the same procedure as described in Example 1, the following compounds were prepared.

(a)

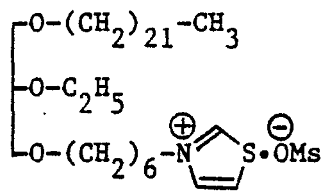

Starting materials: the mesyloxy compound prepared in Reference Example 22(a) and thiazole;

TLC(ethyl acetate:acetic acid:water=3:1:1): Rf= 0.72;

NMR: $\delta$= 10.98(1H,m), 8.22(2H,m), 4.82-4.66(2H,m), 3.72-3.35(11H,m), 2.82(3H,s), 2.10-1.90(2H,m);

IR: $\nu$= 3400, 2930, 2850, 1470, 1110 $cm^{-1}$.

(b)

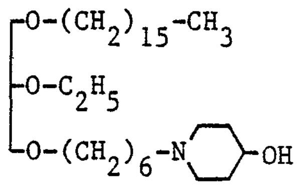

Starting materials: the mesyloxy compound prepared in Reference Example 22(b) and 4-hydroxypiperidine;

TLC(ethyl acetate:acetic acid:water=3:1:1): Rf= 0.48;

MS: m/e=527($M^+$), 499, 483.

(c)

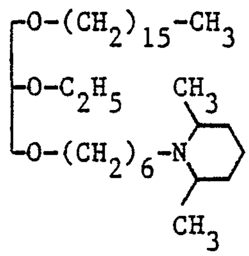

Starting materials: the mesyloxy compound prepared in Reference Example 22(b) and 2,6-dimethylpiperidine;

TLC(ethyl acetate:acetic acid:water=3:1:1): Rf= 0.55;

MS: m/e=539($M^+$), 525.

(d)

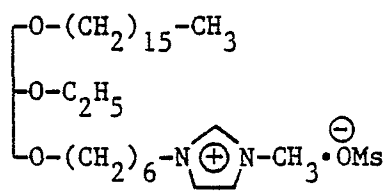

Starting materials: the mesyloxy compound prepared in Reference Example 22(b) and 1-methylimidazole;

TLC(ethyl acetate:acetic acid:water=3:1:1): Rf= 0.50;

NMR: $\delta$= 10.22(1H,m), 7.3-7.18(2H,m), 4.28(2H,t)

4.07(3H,s), 3.7-3.3(11H,m), 2.80(3H,s);

IR: ν= 3500, 2940, 2870, 1580, 1470, 1200, 1120cm$^{-1}$.

(e)

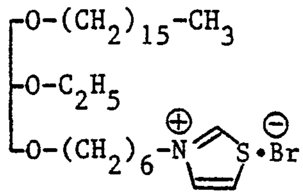

Starting materials: the bromo compound prepared in Reference Example 18(c) and thiazole;

TLC(ethyl acetate:acetic acid:water=3:1:1): Rf= 0.71;

NMR: δ= 11.28(1H,m), 8.42(1H,m), 8.3(1H,m), 4.85(2H,t),
3.7-3.3(11H,m);

IR: ν= 3400, 2930, 2850, 1545, 1460, 1370, 1110cm$^{-1}$.

(f)

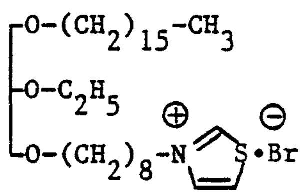

Starting materials: the bromo compound prepared in Reference Example 18(d) and thiazole;

TLC(ethyl acetate:acetic acid:water=3:1:1): Rf= 0.75;

NMR: δ= 11.3(1H,m), 8.36(1H,m), 8.3(1H,m), 4.85(2H,t),
3.72-3.36(11H,m);

IR: ν= 3400, 2920, 2850, 1540, 1460, 1370, 1110cm$^{-1}$.

Example 13

By the same procedure as described in Example 2, the following compounds were prepared.

(a)

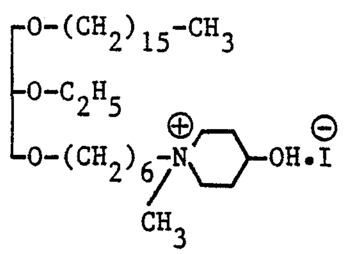

Starting materials: the piperidino compound prepared in Example 12(b) and methyl iodide;

TLC(ethyl acetate:acetic acid:water=3:1:1): Rf= 0.40 and 0.44;

NMR($CDCl_3$+$CD_3OD$ solution): $\delta$= 4.1-4.0(1H,m), 3.8-3.3(17H,m), 3.12-3.10(3H,s), 2.2-1.1(43H,m), 0.86(3H,m);

IR: $\nu$= 3350, 2900, 2850, 1460, 1100 $cm^{-1}$;

MS: m/e= 527, 508, 498, 482.

(b)

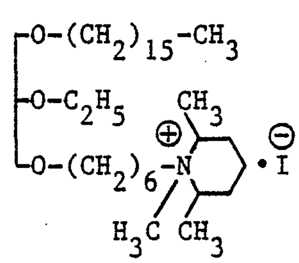

Starting materials: the piperidino compound prepared in Example 12(c) and methyl iodide;

TLC(ethyl acetate:acetic acid:water=3:1:1): Rf= 0.44;

NMR($CDCl_3$+$CD_3OD$ solution): $\delta$= 3.8-3.3(15H,m), 2.87(3H,s), 2.0-1.1(51H,m), 0.86(3H,m);

IR: $\nu$= 3500, 2930, 2850, 1470, 1110 $cm^{-1}$;

MS: m/e=539, 524, 484.

Example 14

Five grams of (2RS)-1-O-hexadecyl-2-O-ethyl-3-O-[7-(3-thiazolio)heptyl]glycerol mesylate, 200 mg of cellulose calcium gluconate (disintegrator), 100 mg of magnesium stearate (lubricant) and 9.7 g of crystalline cellulose were mixed and punched out in a usual manner to obtain tablets each containing 50 mg of the active ingredient.

CLAIMS:

1. A glycerol derivative of the general formula:

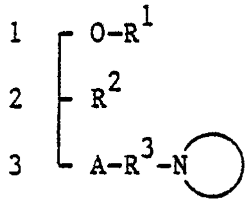

(I)

[wherein A represents an oxygen atom or a carbonyloxy group (wherein the carbonyl group should be attached to a group $R^3$ and the oxa group should be attached to the carbon atom at the third position of the glycerol skeleton), $R^1$ represents an alkyl group of 6 to 22 carbon atoms, or an alkyl group of 2 to 5 carbon atoms being substituted by a phenyl group,
$R^2$ represents a hydroxy group, an amino group, an azido group, an alkyl group of 1 to 20 carbon atoms, an alkoxy group of 1 to 20 carbon atoms, an alkenyl group of 2 to 20 carbon atoms, an alkenyloxy group of 3 to 20 carbon atoms, or a group of the formula: 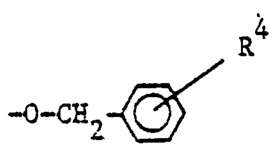 , $-OCOR^5$, $-OCONHR^5$, $-NHCOR^5$, $-NHCOOR^5$ or $-NHCONHR^5$, in which $R^4$ represents a hydrogen atom, a halogen atom, an alkyl group of 1 to 4 carbon atoms or an alkoxy group of 1 to 4 carbon atoms and $R^5$ represents an alkyl group of 1 to 10 carbon atoms or a phenyl group, $R^3$ represents an alkylene group of 1 to 12 carbon atoms and 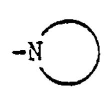 represents

a heterocyclic group, with the proviso that, when A represents the carbonyloxy group, $R^2$ does not represent an amino group], or non-toxic acid addition salts thereof.

2. A compound according to claim 1, wherein $R^1$ is an alkyl group of 14 to 22 carbon atoms.

3. A compound according to claim 2, wherein $R^1$ is a hexadecyl group.

4. A compound according to claim 1, wherein a heterocyclic group represented by 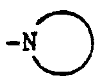 means such a heterocyclic group as meets the following requirements:

(1) the heterocyclic group consists of 4 to 10 atoms,

(2) the group consists of (i) one nitrogen atom, or one nitrogen atom and other one or two nitrogen, oxygen or sulfur atom (with the proviso that the total number of hetero atoms is up to 3), and (ii) at least two carbon atoms,

(3) the group is single ring or bridged bicyclo ring,

(4) the group has or does not have double bond(s) in the ring, and is aromatic or non-aromatic ring,

(5) the group may be substituted by 1 to 5 alkyl groups of 1 to 6 carbon atoms at any optional position in the ring, and

(6) the group may be substituted by 1 or 2 hydroxy groups at any optional position in the ring, when

$R^2$ represents a hydroxy group, an amino group, an azido group, an alkyl group of 1 to 20 carbon atoms, an alkoxy

group of 1 to 20 carbon atoms, an alkenyl group of 2 to 20 carbon atoms, an alkenyloxy group of 3 to 20 carbon atoms, or a group of the formula: $-O-CH_2-$ 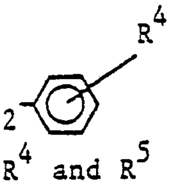 , $-NHCOR^5$, $-NHCOOR^5$ or $-NHCONHR^5$, in which $R^4$ and $R^5$ are as hereinbefore defined.

5. A compound according to claim 4, wherein a heterocyclic group represented by -N 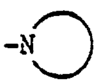 is 1-pyrrolidinyl, piperidino, hexamethyleneimino, 1-piperazinyl, 1-imidazolyl, 1-pyridinio, 1-quinuclidinio, triethylenediamin-1-io, morpholino or 3-thiazolio group, or N-alkyl substituted groups thereof, or hydroxy-substituted group thereof.

6. A compound according to Claim 1, wherein $R^2$ represents a hydroxy group, an amino group, an azido group, or an alkyl or alkoxy group of 1 to 6 carbon atoms, or a group of the formula $-O-CH_2$ 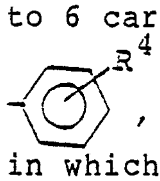 , $-OCOR^5$, $-NHCOR^5$, $-NHCOOR^5$ or $-NHCONHR^5$, in which $R^4$ and $R^5$ are as hereinbefore defined.

7. A compound according to claim 6 which is (2RS)-1-O-hexadecyl-3-O-[7-(1-pyrrolidinyl)heptyl]glycerol (2RS)-1-O-hexadecyl-3-O-[7-(N-methyl-1-pyrrolidinio)heptyl]-glycerol iodide, (2RS)-1-O-hexadecyl-2-dehydroxy-2-amino-3-O-[7-(1-pyrrolidinyl)heptyl]glycerol, (2RS)-1-O- hexadecyl-2-dehydroxy-2-azido-3-O-[7-(1-pyrrolidinyl)heptyl]- glycerol, (2RS)-1-O-hexadecyl-2-O-ethyl-3-O-[7-(3-thiazolio)heptyl]glycerol mesylate, (2RS)-1-O-hexadecyl-2-O-ethyl-3-O-[8-(3-thiazolio)octanoyl]-glycerol mesylate, (2RS)-1-O-hexadecyl-2-O-ethyl-3-O-[6-(3-thiazolio)hexyl]glycerol bromide, (2RS)-1-O-hexadecyl-2-O-ethyl-3-O-[8-(3-thiazolio)octyl]glycerol bromide, (2RS)-1-O-hexadecyl-2-O-ethyl-3-O-[6-(1-methyl-3-imidazolio)hexyl]glycerol mesylate, (2RS)-1-O-hexadecyl-2-O-ethyl-3-O-[6-(4-hydroxypiperidino)hexyl]-glycerol, (2RS)-1-O-hexadecyl-2-O-ethyl-3-O-[6-(N-methyl-4-hydroxypiperidinio)hexyl]glycerol iodide, (2RS)-1-O-hexadecyl-2-O-ethyl-3-O-[6-(2,6-dimethylpiperidino)-hexyl]glycerol, (2RS)-1-O-hexadecyl-2-O-ethyl-3-O-[6-(N-methyl-2,6-dimethylpiperidinio)hexyl]glycerol iodide, (2RS)-1-O-docosyl-2-O-ethyl-3-O-[6-(3-thiazolio)hexyl]glycerol mesylatae, (2RS)-1-O-hexadecyl-2-O-propyl-3-O-[7-(3-thiazolio)heptyl]glycerol mesylate, (2RS)-1-O-hexadecyl-2-O-benzyl-3-O-[4-(1-pyrrolidinyl)butyl]-glycerol, (2RS)-1-O-hexadecyl-2-O-benzyl-3-O-[4-(N-methyl-1-pyrrolidinio)-butyl]glycerol iodide, (2RS)-1-O-hexadecyl-2-O-benzyl-3-O-(4-piperidinobutyl)glycerol, (2RS)-1-O-hexadecyl-2-O-benzyl-3-O-[4-(N-methyl-piperidinio)-butyl]glycerol iodide,

(2RS)-1-O-hexadecyl-2-O-benzyl-3-O-[4-(hexamethyleneimino)-butyl]glycerol,

(2RS)-1-O-hexadecyl-2-O-benzyl-3-O-[4-(N-methyl-hexamethyleneiminio)butyl]glycerol iodide,

(2RS)-1-O-hexadecyl-2-O-benzyl-3-O-[4-(1-piperazinyl)butyl]-glycerol,

(2RS)-1-O-hexadecyl-2-O-benzyl-3-O-[4-(N,N',N'-trimethyl-1-piperazinio)butyl]glycerol iodide,

(2RS)-1-O-hexadecyl-2-O-benzyl-3-O-(4-morpholinobutyl)glycerol,

(2RS)-1-O-hexadecyl-2-O-benzyl-3-O-[4-(N-methylmorpholinio)-butyl]glycerol iodide,

(2RS)-1-O-hexadecyl-2-O-benzyl-3-O-[4-(1-quinuclidinio)butyl]-glycerol mesylate,

(2RS)-1-O-hexadecyl-2-O-benzyl-3-O-[4-(triethylenediamin-1-io)-butyl]glycerol mesylate,

(2RS)-1-O-hexadecyl-2-O-benzyl-3-O-[4-(1-methyl-3-imidazolio)-butyl]glycerol mesylate,

(2RS)-1-O-hexadecyl-2-O-benzyl-3-O-[7-(1-pyrrolidinyl)heptyl]-glycerol,

(2RS)-1-O-hexadecyl-2-O-benzyl-3-O-[7-(N-methyl-1-pyrrolidinio)-heptyl]glycerol iodide, (2RS)-1-O-hexadecyl-2-O-benzyl-3-O-[7-(3-thiazolio)heptyl]- glycerol mesylate, (2RS)-1-O-hexadecyl-2-O-acetyl-3-O-[7-(N-methyl-1-pyrrolidinio)heptyl]-glycerol iodide, (2RS)-1-O-hexadecyl-2-dehydroxy-2-acetylamino-3-O-[7-(1-pyrrolidinyl)-heptyl]glycerol, (2RS)-1-O-hexadecyl-2-dehydroxy-2-acetylamino-3-O-[7-(N-methyl-1-pyrrolidinio)heptyl]glycerol iodide,

(2RS)-1-O-hexadecyl-2-dehydroxy-2-isobutoxycarbonylamino-3-O-[4-(1-pyrrolidinyl)butyl]glycerol,

(2RS)-1-O-hexadecyl-2-dehydroxy-2-isobutoxycarbonylamino-3-O-[4-(N-methyl-1-pyrrolidinio)butyl]glycerol iodide,

(2RS)-1-O-hexadecyl-2-dehydroxy-2-isobutoxycarbonylamino-3-O-[4-(piperidino)butyl]glycerol,

(2RS)-1-O-hexadecyl-2-dehydroxy-2-isobutoxycarbonylamino-3-O-[4-(N-methyl-piperidinio)butyl]glycerol iodide,

(2RS)-1-O-hexadecyl-2-dehydroxy-2-isobutoxycarbonylamino-3-O-[4-(1-quinuclidinio)butyl]glycerol mesylate,

(2RS)-1-O-hexadecyl-2-dehydroxy-2-isobutoxycarbonylamino-3-O-[4-(3-thiazolio)butyl]glycerol mesylate,

(2RS)-1-O-hexadecyl-2-dehydroxy-2-isobutoxycarbonylamino-3-O-[4-(1-pyridinio)butyl]glycerol mesylate,

(2RS)-1-O-hexadecyl-2-dehydroxy-2-isobutoxycarbonylamino-3-O-[4-(1-methyl-3-imidazolio)butyl]glycerol mesylate,

(2RS)-1-O-hexadecyl-2-dehydroxy-2-isobutoxycarbonylamino-3-O-[7-(1-pyrrolidinyl)heptyl]glycerol,

(2RS)-1-O-hexadecyl-2-dehydroxy-2-isobutoxycarbonylamino-3-O-[7-(N-methyl-1-pyrrolidinio)heptyl]glycerol iodide,

(2RS)-1-O-hexadecyl-2-dehydroxy-2-isobutoxycarbonylamino-3-O-[7-(3-thiazolio)heptyl]glycerol mesylate,

(2RS)-1-O-hexadecyl-2-dehydroxy-2-isobutoxycarbonylamino-3-O-[7-(1-pyridinio)heptyl]glycerol mesylate, (2RS)-1-O-hexadecyl-2-dehydroxy-2-(N'-methylureido)-3-O-[7-(1-pyrrolidinyl)heptyl]glycerol or (2RS)-1-O-hexadecyl-2-dehydroxy-2-(N'-methylureido)-3-O-[7-(N-methyl-1-pyrrolidinio)-heptyl]glycerol iodide.

8. A process for the preparation of compounds of the general formula:

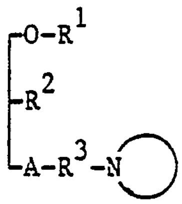

(I)

[wherein A represents an oxygen atom or a carbonyloxy group (wherein the carbonyl group should be attached to a group $R^3$ and the oxa group should be attached to the carbon atom at the third position of the glycerol skeleton), $R^1$ represents an alkyl group of 6 to 22 carbon atoms, or an alkyl group of 2 to 5 carbon atoms being substituted by a phenyl group, $R^2$ represents a hydroxy group, an amino group, an azido group, an alkyl group of 1 to 20 carbon atoms, an alkoxy group of 1 to 20 carbon atoms, an alkenyl group of 2 to 20 carbon atoms, an alkenyloxy group of 3 to 20 carbon atoms, or a group of the formula: $-O-CH_2-$ 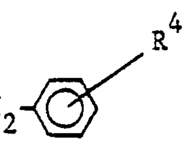 , $-OCOR^5$, $-OCONHR^5$, $-NHCOR^5$, $-NHCOOR^5$ or $-NHCONHR^5$, in which $R^4$ represents a hydrogen atom, a halogen atom, an alkyl group of 1 to 4 carbon atoms or an alkoxy group of 1 to 4 carbon atoms and $R^5$ represents an alkyl group of 1 to 10 carbon atoms or a phenyl group, $R^3$ represents an alkylene group of 1 to 12 carbon atoms and 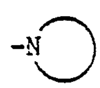 represents a heterocyclic group, with the proviso that, when A represents the carbonyloxy group, $R^2$ does not represent an amino group],

(A) which comprises the reaction of a compund of the general formula:

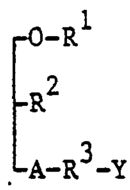

(II)

(wherein Y represents a halogen atom, an alkylsulfonyloxy group or a substituted or unsubstituted arylsulfonyloxy group, and the other symbols are as hereinbefore defined) with a tertiary amine of the general formula:

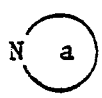

(IIIa)

(wherein 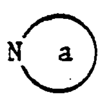 represents a heterocyclic group having tertiary amine(s) in the ring _per se_ or having tertiary amine(s) by substituting nitrogen atom(s) in the ring by alkyl group(s)) and then, if desired, performing salt-exchanging reaction to obtain a compound of the general formula:

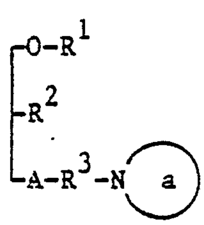

(IA)

(wherein 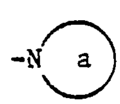 represents a heterocyclic group having quaternary ammonium ion(s) in the ring _per se_ or having

0146258

quaternary ammonium ion(s) by substituting nitrogen atom(s) in the ring by alkyl group(s), and the other symbols are as hereinbefore defined), or

(B) which comprises:

(B-1) the reaction of a compound of the general formula:

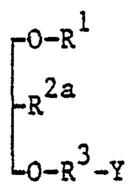

(IIA)

(wherein $R^{2a}$ represents a hydroxy group, an amino group, an azido group, an alkyl group of 1 to 20 carbon atoms, an alkoxy group of 1 to 20 carbon atom, an alkenyl group of 2 to 20 carbon atoms, an alkenyloxy group of 3 to 20 carbon atoms, or a group of the formula: $-O-CH_2-$ 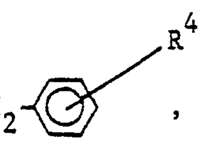 , $-OCONHR^5$, $-NHCOR^5$, $-NHCOOR^5$ or $-NHCONHR^5$, in which $R^4$ and $R^5$ are as hereinbefore defined, and the other symbols are as hereinbefore defined) with a secondary amine of the general formula:

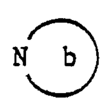

(IIIb)

(wherein 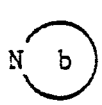 represents a heterocyclic group having secondary amine(s)),

(B-2) the esterification of a compound of the general formula:

0146258

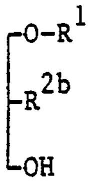 (IV)

,

(wherein $R^{2b}$ represents an azido group, an alkyl group of 1 to 20 carbon atoms, an alkoxy group of 1 to 20 carbon atoms, an alkenyl group of 2 to 20 carbon atoms, an alkenyloxy group of 3 to 20 carbon atoms, or a group of the formula: 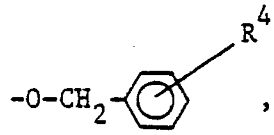 , $-NHCOR^5$, $-NHCOOR^5$ or $-NHCONHR^5$, in which $R^4$ and $R^5$ are as hereinbefore defined, and $R^1$ is as hereinbefore defined) with an acid of the general formula:

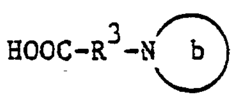 (V)

(wherein 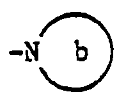 represents a heterocyclic group having tertiary amine(s) and $R^3$ is as hereinbefore defined) or a reactive derivative thereof,

(B-3) reactive elimination of the benzyl group in a compound of the general formula:

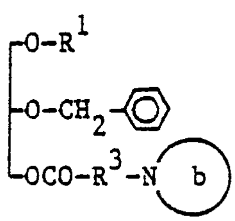 (IB-21)

(wherein the various symbols are as hereinbefore defined),

(B-4) the esterification of a compound of the general formula:

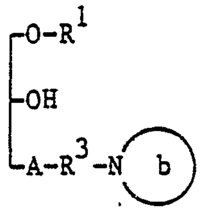

(IB-4)

(wherein the various symbols are as hereinbefore defined) with an acid of the general formula:

$$HOOC-R^5 \qquad (VI)$$

(wherein $R^5$ is as hereinbefore defined) or a reactive derivative thereof,

(B-5) the reaction of a compound of the general formula:

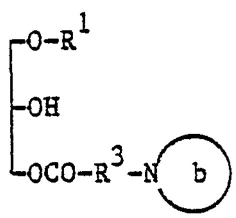

(IB-3)

(wherein the various symbols are as hereinbefore defined) with an isocyanate of the general formula:

$$O=C=N-R^5 \qquad (VII)$$

(wherein $R^5$ is as hereinbefore defined), to obtain a compound of the general formula:

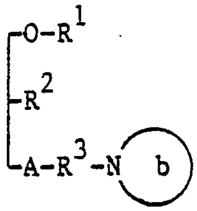

(IB)

(wherein the various symbols are as hereinbefore defined) and, if desired, performing the alkylation of a compound of the general formula (IB), and then, if desired, performing salt-exchainging reaction of a salt obtained by alkylation, or

(C) which comprises:

(C-1) the reduction of a compound of the general formula:

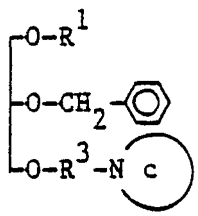

(ID)

or

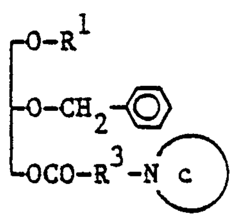

(IE)

(wherein 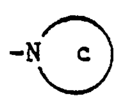 is as hereinbefore defined for 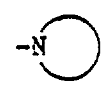 in claim 1, with the proviso that the group should not be substituted by hydroxy group(s), and the other symbols are as hereinbefore defined),

(C-2) the esterification of a compound of a compound of the general formula:

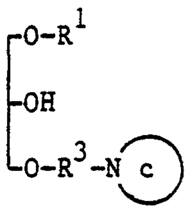

(IC-1)

or

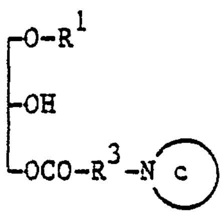

(IC-9)

(wherein the various symbols are as hereinbefore defined) with an acid of the general formula:

$HOOC-R^5$ (VI)

(wherein $R^5$ is as hereinbefore defined) or a reactive derivative thereof,

(C-3) the reaction of a comound of the general formula (IC-1) or (IC-9) with an isocyanate of the general formula:

$O=C=N-R^5$ (VII)

(wherein $R^5$ is as hereinbefore defined),

(C-4) the reaction of a compound of the general formula:

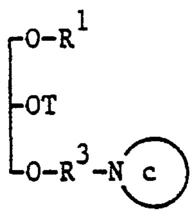

(XLIV)

(wherein T represents an alkylsulfonyl group or a substituted or unsubstituted arylsulfonyl group and the other symbols are as hereinbefore defined) with sodium azide,

(C-5) the reduction of a compound of the general formula:

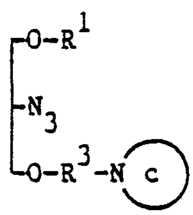

(IC-4)

(wherein the various symbols are as hereinbefore defined),

(C-6) the reaction of a compound of the general formula:

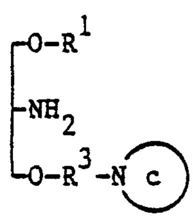

(IC-5)

(wherein the various symbols are as hereinbefore defined) with a compound of the general formula:

$X^2$-CO-$R^5$ (XLV)

or

$R^5$-CO-O-CO-$R^5$ (XLVI)

(wherein $X^2$ represents a halogen atom and $R^5$ is as hereinbefore defined),

(C-7) the reaction of a compound of the general formula (IC-5) with a compound of the general formula:

$X^3$-COOR$^5$ (XLVII)

(wherein $X^3$ represents a halogen atom and $R^5$ is as hereinbefore defined) or

(C-8) the reaction of a compound of the general formula (IC-5) with a compound of the general formula (VII), to obtain a compound of the general formula:

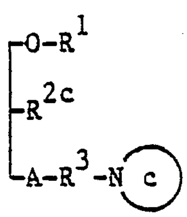

(IC)

(wherein (i) when A is an oxygen atom $R^{2c}$ represents a hydroxy group, an amino group, an azido group, or a group of the formula: -OCOR$^5$, -OCONHR$^5$, -NHCOR$^5$, -NHCOOR$^5$ or -NHCONHR$^5$, in

which $R^5$ is as hereinbefore defined, or (ii) when A is a carbonyloxy group, $R^{2c}$ represents a hydroxy group, or a group of the formula: $-OCOR^5$ or $-OCONHR^5$, in which $R^5$ is as hereinbefore defined, and the other symbols are as hereinbefore defined).

9. A platelet aggregation inhibiting, anti-asthmatic, anti-allergic, anti-inflammatory, hypotensive or anti-tumour pharmaceutical composition which comprises, as active ingredient, an effective amount of at least one compound of the general formula (I) depicted in claim 1, wherein the various symbols are as defined in claim 1, or a non-toxic acid addition salt thereof, together with a pharmaceutical carrier or coating.

10. A method of inhibiting platelet aggregation, or for the prevention and treatment of asthma, allergy, inflammation, hypertension and tumour which comprises the oral or parenteral administration of an effective amount of a compound as claimed in claim 1, wherein the various symbols are as defined in claim 1, or a non-toxic acid addition salt thereof.